# EUROPÄISCHE PATENTSCHRIFT

(11) **EP 1 663 915 B1**
(45) Veröffentlichungstag und Bekanntmachung des Hinweises auf die Patenterteilung: **02.12.2009**
(21) Anmeldenummer: 04765027.0
(22) Anmeldetag: 10.09.2004
(51) Int. Cl.: C07B 59/00, C07C 227/14, C07C 229/36

(54) **HERSTELLUNG VON [18 F] FLUORMARKIERTEN AROMATISCHEN L-AMINOSÄUREN**
METHOD FOR THE PRODUCTION OF [18F] FLUORIDE-MARKED AROMATIC L-AMINO ACIDS
PRODUCTION DE L-AMINOACIDES [18F] FLUOROMARQUES

(30) Priorität: 25.09.2003 DE 10346228
(43) Veröffentlichungstag der Anmeldung: 07.06.2006
(73) Patentinhaber: Eberhard-Karls-Universität Tübingen Universitätsklinikum, 72076 Tübingen (DE)
(72) Erfinder: MACHULLA, Hans-Jürgen, 72072 Tübingen (DE); ÜBELE, Michael, 72070 Tübingen (DE); BLOCHER, Achim, 79639 Grenzach-Whylen (DE)
(74) Vertreter: Findeisen, Marco
(86) Internationale Anmeldenummer: PCT/EP2004/010094
(87) Internationale Veröffentlichungsnummer: WO 2005/037737

(56) Entgegenhaltungen:
- WO-A-02/44144
- WO-A-94/00460

## Beschreibung

Die vorliegende Erfindung betrifft ein Verfahren zur Herstellung [¹⁸F]fluormarkierter aromatischer L-Aminosäuren, ein Verfahren zur Herstellung eines [¹⁸F]fluormarkierten Diagnostikums, ein Verfahren zur Visualisierung von Stoffwechselvorgängen, sowie die Verwendung einer L-enantiomeren Verbindung zur Herstellung von [¹⁸F]fluormarkierten aromatischen L-Aminosäuren.

Radioaktiv markierte Aminosäuren finden in verschiedensten Gebieten der Naturwissenschaft, insbesondere in den Biowissenschaften, und auch in der Medizin Anwendung. Dabei handelt es sich vor allem um sogenannte "Tracer", d.h. um solche Aminosäuren, mit denen sich biologische, biochemische, chemische o.ä. Vorgänge verfolgen lassen. Die Markierung der Aminosäuren erfolgt hierbei durch den Einbau von kurzlebigen Radionukliden (Radioindikatoren oder Leitisotope). Ein Beispiel für ein derartiges Radionuklid ist [18F]Fluor. Die radioaktiv markierten Aminosäuren bzw. Tracer können so konstruiert werden, dass sich diese hinsichtlich des zu untersuchenden Vorgangs genau so wie unmarkierte Substanzen verhalten. Darüber hinaus ist auch eine gezielte Änderung der Eigenschaften des Tracers gegenüber denen der entsprechend unmarkierten Substanz möglich, so dass es bspw. zu keiner oder zu einer präzise stark beschränkten Metabolisierung des Tracers ("metabolic trapping") kommt, wenn dieser in einen lebenden Organismus eingebracht wird.

Derartige radioaktiv markierte Aminosäuren werden bspw. als Tracer in der Positron-Emissions-Tomographie (PET) eingesetzt. Hierbei handelt es sich um ein szintigraphisches Diagnoseverfahren, das die Vorteile tomographischer Schichtaufnahmen mit der selektiven Darstellung physiologischer Stoffwechselfunktionen in sich vereint. Mittels PET gelingt die Darstellung essentieller Stoffwechselvorgänge *in vivo.* Die bei der PET eingesetzten Tracer setzen am Ort ihrer *in-vivo* biologischen Wirkung bei deren Zerfall Positronen frei, was letztlich die Aussendung von Gammastrahlen zur Folge hat. Diese radioaktive Strahlung kann mit Hilfe eines PET-Scanners sowohl qualitativ als auch quantitativ detektiert werden und somit bspw. Rückschlüsse auf Veränderungen im Stoffwechselsystem eines Patienten ermöglichen.

Die wichtigsten diagnostischen Einsatzgebiete von PET betreffen die Untersuchung von lokalem Blutfluss und Sauerstoffverbrauch, Substratstoffwechsel, Enzymkonzentrationen, sowie der Biochemie neuronaler und hormoneller Transmitter, insbesondere Rezeptordichten und -besetzungen sowie von Immunreaktionen. Ferner können mittels PET auch molekulare Vorgänge der Proteinbiosynthese untersucht werden.

Zunehmend findet die PET auch Anwendung in der Diagnostik und unterstützenden Therapiekontrolle bei Herz- und Krebserkrankungen sowie neurodegenerativen Erkrankungen, wie bspw. Morbus Parkinson, Morbus Alzheimer, Demenz.

Ein weiterer interessanter Ansatz besteht auch auf dem Gebiet der Pharmakologie, indem pharmakologisch wirksame Aminosäuren radioaktiv markiert werden und hinsichtlich ihrer Verteilung am Wirkort mit PET quantifiziert und bezüglich ihrer Wirksamkeit geprüft werden können.

Insbesondere durch die jährlich stark steigende Anzahl der PET-Zentren weltweit (270 Zentren im Jahre 2001), als auch deren zunehmende medizinisch-wissenschaftliche Akzeptanz besteht ein erhöhter Bedarf an Tracern, bspw. an [¹⁸F]fluormarkierten aromatischen L-Aminosäuren.

Im Stand der Technik sind verschiedene Verfahren zur Herstellung [¹⁸F]fluormarkierter aromatischer L-Aminosäuren 1 bekannt. Das gängigste Verfahren ist dabei die elektrophile Substitution via [¹⁸F] Fluorgas ([¹⁸F]F₂). Bei dieser Synthese wird die Fluordestannylierung des Precursors 2 als elektrophile Substitutionsreaktion, bei der [¹⁸F]F₂ eingesetzt wird, durchgeführt:

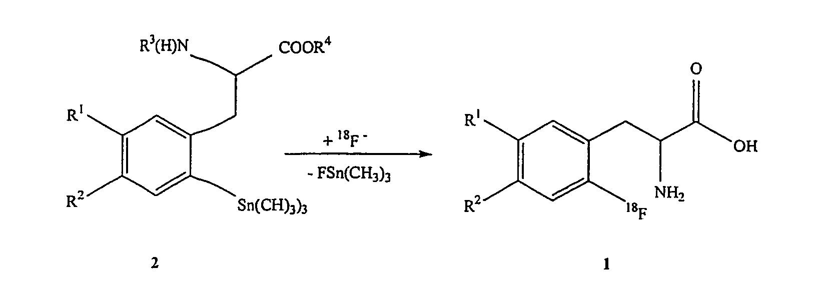

Dieses bekannte Verfahren ist bspw. in A. Luxen, M. Guillaume, W. P. Melega, V. W. Pike, O. Solin, R. Wagner, Nucl. Med. Biol, Vol. 19, No. 2, 14 9-158 (1992) beschrieben.

Dieses Verfahren weist eine Reihe von erheblichen Nachteilen auf. [¹⁸F]F₂ wird über die Bestrahlung von Neon hergestellt, wobei dem Neon 0,1% [¹⁹F] F₂ beigemischt wird. Aus diesem Grunde erhält man bei der elektrophilen Substitution geträgerte markierte aromatische L-Aminosäuren, d.h. solche, die mit nichtradioaktivem Fluor markiert sind. Ferner können nur 50 % der Fluor-18-Atome genutzt werden, die andere Hälfte reagiert mit der Abgangsgruppe. Hinzu kommt, dass lange Neonbestrahlungszeiten erforderlich sind und es lediglich zu einer geringen Ausbeute an [¹⁸F]F₂ kommt.

Eine weitere wichtige Methode betrifft die nucleophile Substitution via [¹⁸F]Fluorid, bei der von unterschiedlichen Vorstufen ausgegangen wird, die in manuell durchgeführten Reaktionen zu den [¹⁸F]fluormarkierten aromatischen L-Aminosäuren umgesetzt werden. 6-[¹⁸F]Fluoro-3,4-dihydroxy-L-phenylalanin (FDOPA) wird bspw. aus den Nitrobenzaldehyd-Derivaten **3** (R¹=R²=OH-Schutzgruppen) hergestellt. Diese werden nach dem Markierungsschritt mit [¹⁸F]Fluorid über eine mehrstufige Synthese zu FDOPA, umgesetzt:

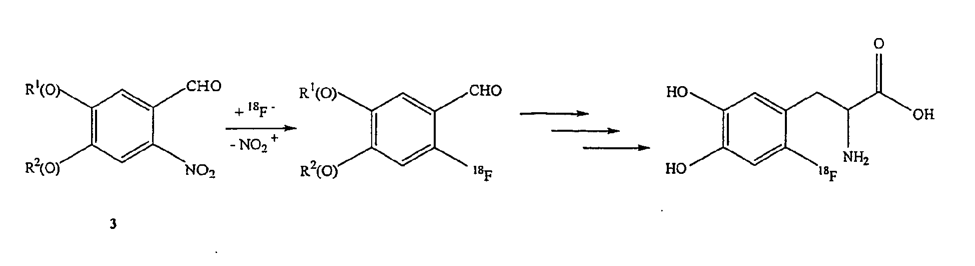

Dieses bekannte Verfahren ist bspw. beschrieben in Lemaire C., Guillaume M., Cantineau R., Christiaens L., J. Nucl. Med. 31, 1247 (1990); Antoni G., Langstrom B., Acta Chem. Scand. 40, 152 (1987); Lemaire C., Guillaume M., Christiaens L., J. Nucl. Med. 30, 752 (1989); Lemaire C., Guillaume M., Cantineau R., Plenevaux A., Christiaens L., J. Label. Compds. Radiopharm. 30, 269 (1991).

Dieses Verfahren hat den entscheidenden Nachteil, dass es eine vielstufige Synthese darstellt, die sehr arbeits-, zeit- und kostenaufwendig ist. Ferner liefert das bekannte Verfahren unzureichende Enantiomerenreinheit. Zusätzlich zu einer Produktabtrennung mittels HPLC-Reinigung ist dort eine Enantiomerentrennung über eine chirale HPLC-Einheit notwendig. Das bekannte Verfahren ist deshalb für eine automatisierte Routineproduktion von [¹⁸F]fluormarkierten L-Aminosäuren nur schlecht geeignet.

Im Stand der Technik sind weitere Verfahren zur Herstellung [¹⁸F]fluormarkierter aromatischer L-Aminosäuren beschrieben. Bspw. werden 2-[¹⁸F]Fluoro-L-phenylalanin (1, R¹=R²=H) oder 2-[¹⁸F]Fluoro-L-tyrosin (1, R¹=H, R²=OH) über eine technisch äußerst problematische Fluordediazotierungsreaktion aus den entsprechenden Triazen-Derivaten 4 (R⁴=Alkyl-Gruppe) oder über eine nucleophile Substitution an den Trimethylammonium-Derivaten **5** hergestellt: 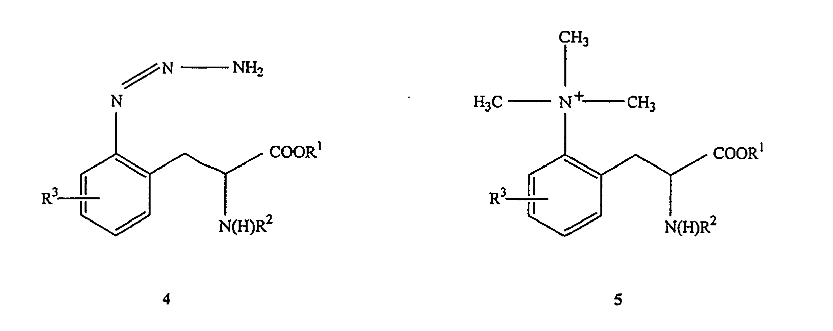 Diese bekannten Verfahren werden ebenfalls in dem Übersichtsartikel von Luxen et al. (a.a.O.) beschrieben. Näheres zu dem Triazen-Derivat **4** ist ferner beschrieben in Thierry Pages, Bernhard R. Langlois, Didier Le Bars, Patricia Landais, J. Fluorine Chem. 107, 329-335 (2001). Das Trialkylammonium-Derivat **5** ist in der WO 02/44144 A2 beschrieben.

Auch diese Verfahren weisen verschiedenste Nachteile auf. So sind die hier beschriebenen Verfahren technisch mit größeren Schwierigkeiten verbunden, so dass eine automatisierte Durchführung nicht möglich ist. Eine Vielzahl an Nebenreaktionen erfordert eine aufwendige Aufreinigung der Produkte. Ferner ist das äußerst interessante FDOPA über die beiden dargestellten Derivate **4** und **5** nicht herstellbar.

Aufgabe der vorliegenden Erfindung ist es deshalb, ein Verfahren zur Herstellung [¹⁸F]fluormarkierter aromatischer L-Aminosäuren bereitzustellen, mittels dem die vorstehend beschriebenen Nachteile vermieden werden. Insbesondere soll ein Verfahren bereitgestellt werden, das sich durch wenige Syntheseschritte, eine kurze Synthesedauer und durch eine hohe Reproduzierbarkeit auszeichnet. Ferner soll ein solches Verfahren einfach zu automatisieren sein.

Diese Aufgabe wird erfindungsgemäß durch die Bereitstellung eines Verfahrens zur Herstellung [¹⁸F]fluormarkierter aromatischer L-Aminosäuren mit folgenden Schritten gelöst:
(1) Bereitstellung der nachfolgend dargestellten L-enantiomeren Verbindung in einem geeigneten Reaktionsmedium: 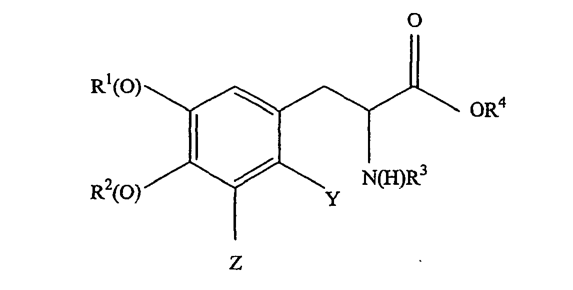 wobei R¹ und R² geeignete Schutzgruppen für OH-Gruppen sind, sofern eine Hydroxyaminosäure hergestellt werden soll; wobei Z eine elektronenziehende Gruppe ist;
   wobei Y eine Abgangsgruppe für eine nucleophile Substitution ist; wobei R³ einer oder mehreren geeigneten Schutzgruppen für eine Aminofunktion entspricht;
   wobei R⁴ einer geeigneten Schutzgruppe für eine Carboxylfunktion entspricht;
(2) nucleophile Substitution von Y durch ein negativ geladenes [¹⁸F]Fluorid-Ion zur Herstellung der nachfolgend dargestellten Verbindung: 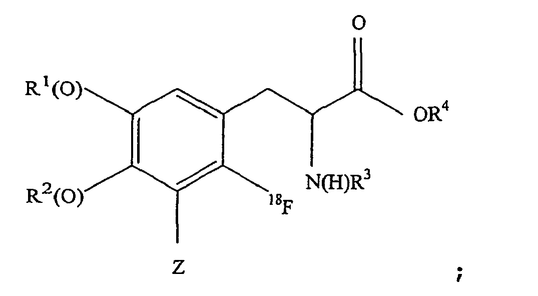
(3) Abspaltung von Z zur Herstellung der nachfolgend dargestellten Verbindung: 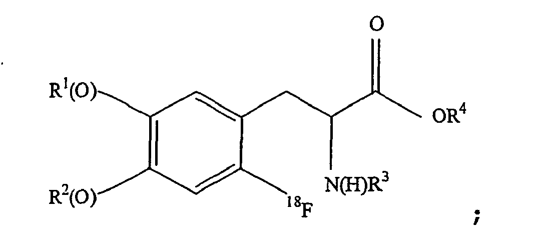
(4) hydrolytische Abspaltung von R³ und R⁴ zur Herstellung der nachfolgend dargestellten Verbindung: 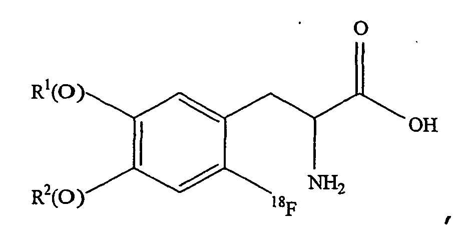 und
(5) hydrolytische Abspaltung von R¹ und R² zur Herstellung der nachfolgend dargestellten [¹⁸F]fluormarkierten aromatischen L-Aminosäure: 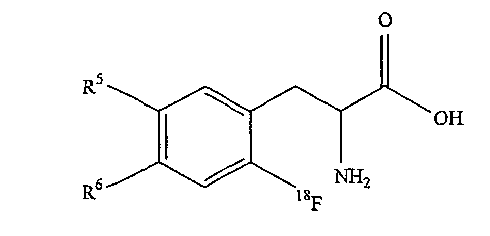 wobei R⁵ und R⁶ Substituenten sind, die jeweils einem H-Atom oder einer OH-Gruppe entsprechen.

Die der Erfindung zu Grunde liegende Aufgabe wird hiermit vollkommen gelöst.

Die Erfinder haben erkannt, dass sich ausgehend von dem in Schritt (1) bereitgestellten L-enantiomerenreinen Markierungsprecursor überraschenderweise in nur wenigen Schritten [¹⁸F]Fluoro-L-phenylalanin-Derivate, d.h. [¹⁸F]fluormarkierte aromatische Aminosäuren wie 2-[¹⁸F]Fluoro-L-phenylalanin (R⁵=R⁶=H), 2-[¹⁸F]Fluoro-L-tyrosin (R⁵=H, R⁶=OH), 2-[¹⁸F]Fluoro-5-hydroxy-L-phenylalanin (R⁵=OH, R⁶=H; 2-[¹⁸F]Fluoro-meta-L-tyrosin) und 6-[¹⁸F]Fluoro-3,4-dihydroxy-L-phenylalanin (R⁵=R⁶=OH; [¹⁸F]DOPA), herstellen lassen. Dabei war besonders überraschend, dass überhaupt ausgehend von bereits chiral reinen, mit Schutzgruppen versehenen L-enantiomeren aromatischen Aminosäuren über trägerfreies [¹⁸F]Fluorid eine direkte Markierung und Umsetzung zur gewünschten L-Aminosäure innerhalb weniger Schritte möglich ist. Im Stand der Technik wird nämlich bislang die Auffassung vertreten, dass eine Markierung stets an einfachen Aminosäure-Vorstufen durchgeführt werden müsse und erst daran anschließend die eigentliche "Konstruktion" der Aminosäure sowie eine Enantiomerentrennung zu erfolgen habe.

In der praktischen Durchführung des erfindungsgemäßen Verfahrens erfolgen die Schritte (4) und (5) gleichzeitig, d.h. die hydrolytische Abspaltung der Schutzgruppen R¹ bis R⁴ erfolgt in einem Schritt. Lediglich zur besseren Darstellung der Herstellung verschiedener markierter Aminosäuren, d.h. von Hydroxy- und Nicht-Hydroxyaminosäuren, wurde die Differenzierung in zwei Schritte gewählt.

Es versteht sich, dass die Schutzgruppen R¹ und R² nur dann vorzusehen sind, sofern Hydroxyaminosäuren, d.h. markiertes Tyrosin (nur R²), Hydroxyphenylalanin (nur R¹) oder DOPA (R¹ und R²), hergestellt werden sollen. Zur Herstellung von Nicht-Hydroxyaminosäuren gilt, dass R^{1/2}(O) durch ein H-Atom ersetzt wird. Das gilt natürlich auch für die Herstellung von markiertem Tyrosin (R¹(O) ersetzt durch H) und markiertem m-Hydroxyphenylalanin (R²(O) ersetzt durch H). Eine hydrolytische Abspaltung der genannten H-Atome in Schritt (5) erübrigt sich in diesen Fällen, d.h. bspw. zur Herstellung von markiertem Phenylalanin entfällt Schritt (5) gänzlich. Im letzteren Fall gilt R¹ (0) =R²(O) =R⁵=R⁶=H.

Vor diesem Hintergrund ist Gegenstand der vorliegenden Erfindung auch die Verwendung des in Schritt (1) des erfindungsgemäßen Verfahrens bereitgestellten L-enantiomerreinen Markierungsprecursors zur Herstellung [¹⁸F]fluormarkierter aromatischer L-Aminosäuren.

Bei der vorliegenden Erfindung handelt es sich folglich um eine Abkehr vom bisherigen Vorgehen im Stand der Technik. Bei dem neuen Verfahren entfällt die zeitaufwendige Enantiomerentrennung, die im Stand der Technik zum Erhalt der markierten L-Aminosäure notwendig ist. Das erfindungsgemäße Verfahren stellt deshalb eine vereinfachte Synthese bereit, wobei maximale stereochemische Reinheit der herzustellenden [¹⁸F]fluormarkierten aromatischen L-Aminosäure gewährleistet ist. Dies ist insbesondere für eine mögliche Verwendung der [¹⁸F]fluormarkierten aromatischen L-Aminosäure in einem lebenden Organismus entscheidend, da nur chiral reine L-enantiomere Aminosäuren in biologische Zellen gelangen und dort ggf. verstoffwechselt werden können.

Das erfindungsgemäße Verfahren ist ferner vollständig automatisierbar, wodurch reproduzierbar eine hohe Produktqualität sichergestellt wird. Die damit verbundene gut kontrollierbare Strahlenemission bedeutet zusätzlich für das Laborpersonal ein Mehr an Sicherheit.

Mittels des erfindungsgemäßen Verfahrens gelingt dabei die zuverlässige Herstellung diverser aromatischer L-Aminosäuren.

Das erfindungsgemäße Verfahren zeichnet sich insbesondere durch seine kurze Synthesedauer aus. Die Herstellung von 6-[¹⁸F]Fluoro-3,4-dihydroxy-L-phenylalanin ([¹⁸F]DOPA) gelingt in einer Zeit von etwa 45 Minuten, wohingegen die Herstellung dieser Aminosäure gemäß nukleophiler Substitution via [¹⁸F]Fluorid nach dem Stand der Technik ca. 110 Minuten benötigt; vgl. hierzu Lemaire C. et al. (1993), Appl. Radiat. Isot., Vol. 44, Nr. 4, 737-744.

Das erfindungsgemäße Verfahren lässt sich in jedem geeigneten im Stand der Technik für Fluoridmarkierungen beschriebenen Reaktionsmedium durchführen, wie bspw. Acetonitril, Dimethylsulfoxid, Benzonitril, Dimethylformamid.

Der in Schritt (1) bereitgestellte L-enantiomere Markierungsprecursor ist mittels allgemein bekannter Methoden der organischen Chemie herstellbar. Für die Substituenten R¹-R⁴ kommen beliebig viele organisch-chemische Schutzgruppen für Hydroxygruppen (R¹, R²), Aminofunktionen (R³) bzw. Carboxyfunktionen (R⁴) in Frage. Diese Schutzgruppen sowie Verfahren zu deren Anbringung bzw. Entfernung sind im Stand der Technik allgemein bekannt; vgl. hierzu bspw. Theodora W. Green, Peter G.M. Wuts, "Protective Groups in Organic Synthesis", 3rd Edition (1999), John Wiley & Sons Inc., ISBN 0-471-160119-9.

Der für das erfindungsgemäße Verfahren entscheidende Markierungsprecursor ist deshalb ebenfalls Gegenstand der vorliegenden Erfindung, wobei Z=CHO und Y=NO₂ entsprechen.

Bei dem erfindungsgemäßen Verfahren bzw. bei der erfindungsgemäßen Verwendung ist es bevorzugt, wenn R¹ und R² jeweils ausgewählt sind aus der Gruppe bestehend aus: CH₃, CH₂OCH₃, CH₂OCH₂(C₆H₅), CH₂SCH₃, CH₂SCH₂(C₆H₅), CH₂CH₂Cl, CH₂OCH₂Br, CH₂COC₆H₃-3,4-Cl, CH₂COC₆H₃-2,6-Cl₂, CH₂=CH₂, CH(CH₃)₂, *c*-C₆H₁₁, C(CH₃)₃, CH₂C₆H₅, 2,6-(CH₃)₂C₆H₃CH₂, 4-CH₃OCH₄CH₂, *o*-NO₂-C₆H₄CH₂, (CH₃)₂NCOC₆H₄CH₂, COCH₃, COC₆H₅, CO₂CH₃, COOCH₂CCl₃, CONHPh (Ph=Phenyl) und CONH-*i*-Bu (Bu=Butyl).

Die Verwendung dieser Schutzgruppen hat den Vorteil, dass es sich hierbei um besonders bewährte und einfach anbringbare und entfernbare Schutzgruppen handelt. Wie erwähnt, bedarf es im Falle der Herstellung von [¹⁸F]fluormarkiertem Tyrosin lediglich der Schutzgruppe R¹ an Position vier des Benzolrings, im Falle von [¹⁸F]fluormarkiertem m-Tyrosin der Schutzgruppe R¹ an Position 5 des Benzolrings und im Falle von [¹⁸F]fluormarkiertem Phenylalanin um keinerlei Schutzgruppen. Bei der Herstellung dieser drei zuvor genannten [¹⁸F]fluormarkierten aromatischen L-Aminosäuren wird lediglich R² (Herstellung von 2-[¹⁸F]Fluoro-L-tyrosin), bzw. R¹ hydrolytisch abgespalten (Herstellung von 2-[¹⁸F]Fluoro- meta-L-tyrosin) bzw. Schritt (5) entfällt ganz (Herstellung von 2-[¹⁸F]Fluoro-L-phenylalanin). Bei der Herstellung von [¹⁸F]DOPA sind sowohl R¹ als auch R² als Schutzgruppen vorzusehen, die in Schritt (5) erfindungsgemäß wieder abgespalten werden.

Die Vielzahl der bevorzugten Schutzgruppen zeigt ferner die vielseitige Anwendbarkeit des Verfahrens auf.

Nach einer weiteren bevorzugten Variante entsprechen R¹ und R² einem cyclischen Acetal, das vorzugsweise ausgewählt ist aus der Gruppe bestehend aus: Methylen-, Dimethylmethylen-, Cyclohexyliden-, Diphenylmethylen-, Ethoxymethylenacetal und cyclischem Borsäureester.

Diese Maßnahme hat den Vorteil, dass es sich hierbei um eine etablierte, einfach anbringbare und entfernbare Schutzgruppe handelt, die insbesondere bei der Herstellung von FDOPA Verwendung finden kann. In dem DOPA-Molekül stehen am Benzolring zwei OH-Gruppen in ortho-Position zueinander. Die Acetal-Schutzgruppe bildet hierbei eine Methylen-Brücke zwischen den beiden OH-Gruppen. Nachstehend ist beispielhaft ein Markierungsprecursor aus Schritt (1) dargestellt, bei dem R¹ und R² einem Methylenacetal entsprechen:

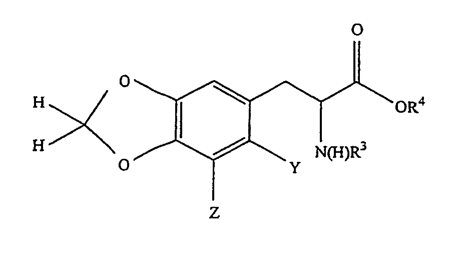

Bei dem erfindungsgemäßen Verfahren bzw. der erfindungsgemäßen Verwendung ist es ferner bevorzugt, wenn Z einem Substituenten zweiter Ordnung entspricht, der vorzugsweise ausgewählt ist aus der Gruppe bestehend aus: CHO, NO₂, SO₂Me (Me=Methyl), NR₃⁺ (R=Alkylgruppe), CF₃, CN, COR (R=Alkyl/Aryl), COOH, Br, Cl und I.

Nach dieser besonderen Ausführungsform sind für Z grundsätzlich sämtliche Substituenten "zweiter Ordnung" geeignet. Dabei dient Z zur Positivierung des zu Z ortho stehenden benachbarten Kohlenstoffatoms mit der Abgangsgruppe Y, wodurch die Anlagerung von negativ geladenem Fluorid an dieses C-Atom erleichtert wird und nach Abgang von Y⁻ die entsprechende Fluorverbindung entsteht. Diese bevorzugte Maßnahme hat deshalb den Vorteil, dass ein Substituent mit ausreichend hohem elektronenziehenden Potenzial bereitgestellt wird, der somit als sog. aktivierende Gruppe gut geeignet ist. Die genannten bevorzugten Substituenten sind schnell und leicht mittels im Stand der Technik bekannter Methoden entfernbar.

Sofern die elektronenziehende Gruppe Z einer Aldehydgruppe (CHO) entspricht, ist es bevorzugt, wenn Schritt (3) mit Hilfe eines Decarbonylierungskatalysators, vorzugsweise mit Tris-(triphenylphosphin)-rhodium(I)-chlorid und/oder konzentrierter Schwefelsäure (H₂SO_{4konz.}) und/oder Palladium auf Aktivkohle (Pd/C) und/oder einem Wilkinson-Katalysator (Rhodium-Katalysator), erfolgt.

Diese Maßnahme hat den Vorteil, dass auf Grund des Decarbonylierungskatalysators eine zielgerichtete Abspaltung der Aldehydgruppe möglich wird, ohne dass es bspw. zur Abspaltung des addierten [¹⁸F] Fluorid-Ions kommt.

Weiter bevorzugt ist es, wenn Y einem Substituenten entspricht, der ausgewählt ist aus der Gruppe bestehend aus: F, NO₂, OTs (= Tosyl/Toluolsulfonsäureester) , Cl, Br, I, N₃ und NR₃⁺ (R=Alkyl/Aryl).

Dabei ist von Vorteil, dass auch diese Maßnahme eine für nucleophile Substitutionen an Aromaten geeignete und im Stand der Technik etablierte Abgangsgruppe mit ausreichend hoher Abspaltungstendenz Verwendung findet.

Bei einer bevorzugten Weiterbildung des erfindungsgemäßen Verfahrens bzw. der erfindungsgemäßen Verwendung entspricht R³ einem oder mehreren Substituenten, die ausgewählt sind aus der Gruppe bestehend aus: 9-Fluorenylmethylcarbamat, CO₂CH₂CCl₃, CO₂CH₂CH₂Ph (Ph=Phenyl), CO₂C(CH₃)CHBr₂, CO₂C(CH₃)₂CCl₃, CO₂C(CH₃)₃, N-Hydroxypiperidinylcarbamat, CO₂CH₂Ph (Ph=Phenyl), CO₂CH₂*-p-*CH₃OC₆H₄, CO₂CH₂*-p*-NO₂C₆H₄, CHO; COCH₃, COCH₂Cl, COCCl₃, COCF₃, COC₆H₅, Phthalimid, Dithiasuccinimid, N-5-Dibenzosuberylamin, N-1,1-Dimethylthiomethylen, N-Benzyliden, N-1,3-Dithiolan-2-yliden, N-Diphenylmethylen, =CHN(CH₃) und =CN(CH₂C₆H₅)₂.

Ferner ist es bevorzugt, wenn R⁴ einem oder mehreren Substituenten entspricht, die ausgewählt sind aus der Gruppe bestehend aus: CH₃, C₂H₅, CH₂OCH₃, CH₂SCH₃, CH₂OCH₂C₆H₅, CH₂CCl₃, C(CH₃)₃, CH₂C₆H₅, CH₂C₆H₂-2,4,6-(CH₃)₃-.

Die beiden vorstehend genannten Maßnahmen haben den Vorteil, dass damit geeignete und im Stand der Technik etablierte Schutzgruppen für die Aminofunktion bzw. Carboxylfunktion Verwendung finden, die insbesondere ausreichend leicht anbringbar und ausreichend leicht abspaltbar sind.

Nach einer bevorzugten Ausgestaltung des erfindungsgemäßen Verfahrens erfolgen sämtliche Schritte in einem einzigen Reaktionsgefäß.

Diese Vorgehensweise hat den Vorteil, dass eine besonders einfache Handhabbarkeit des Verfahrens erreicht wird, wobei die Kontamination von weiteren Reaktionsgefäßen vermieden wird. Dies bedeutet zusätzlich hohe Sicherheit für das Laborpersonal. Diese Maßnahme ermöglicht ferner eine einfache Automatisierbarkeit des erfindungsgemäßen Verfahrens.

Nach einer bevorzugten Weiterbildung des erfindungsgemäßen Verfahrens erfolgen sämtliche Schritte automatisiert, vorzugsweise mit Hilfe eines Syntheseroboters, weiter bevorzugt mit Hilfe eines kompakten Syntheseautomaten.

Diese Maßnahme ermöglicht auf vorteilhafte Art und Weise die Ausgestaltung des erfindungsgemäßen Verfahrens als Hochdurchsatzverfahren, so dass dieses für die Routineproduktion von [¹⁸F]fluormarkierten aromatischen L-Aminosäuren verwendbar ist. Dadurch wird ferner eine hohe Produktqualität und Reproduzierbarkeit sichergestellt. Die Verwendung eines kompakten Syntheseautomaten hat dabei den zusätzlichen Vorteil, dass dieser im Vergleich zu üblichen Syntheserobotern weitaus weniger Platz einnimmt, was die Kosten bspw. für eine Bleizelle bzw. ein radiochemisches Labor deutlich reduziert.

Bei einer bevorzugten Weiterbildung des erfindungsgemäßen Verfahrens wird ein weiterer folgender Schritt durchgeführt:
(6) Herstellung eines Tracers durch Formulierung der [¹⁸F]fluormarkierten aromatischen L-Aminosäure mit einem pharmazeutisch akzeptablen Träger und/oder Lösungsmittel.

Diese Weiterbildung hat zum Vorteil, dass die radioaktiv markierte Aminosäure bereits in solch einer Form bereitgestellt wird, dass diese bspw. bei der Verwendung im Rahmen der eingangs genannten PET direkt in einen lebenden Organismus appliziert werden kann. Pharmazeutisch akzeptable Träger bzw. Lösungsmittel sind im Stand der Technik allgemein bekannt; vgl. Bauer, Frömming, Führer, Lehrbuch der pharmazeutischen Technologie, 6. Auflage, 1999, Wiss. Verl.-Ges., Stuttgart.

Vor dem Hintergrund des eingangs und vorstehend diskutierten Potenzials der erfindungsgemäß hergestellten [¹⁸F]fluormarkierten aromatischen L-Aminosäure und der damit verbundenen Vorteile ist Gegenstand der vorliegenden Erfindung auch ein Verfahren zur Herstellung eines Diagnostikums mit folgenden Schritten:
(1) Bereitstellung einer [¹⁸F]fluormarkierten aromatischen L-Aminosäure, und
(2) Formulierung der Aminosäure aus Schritt (1) mit einem pharmazeutisch akzeptablen Träger und/oder Lösungsmittel sowie ggf. weiterer pharmazeutischer Hilfsstoffe,
wobei Schritt (1) mittels des vorstehend beschriebenen erfindungsgemäßen Verfahrens erfolgt. Dabei ist es bevorzugt, wenn das Diagnostikum zur Verwendung im Rahmen der Positron-Emissions-Tomographie (PET) vorgesehen ist.

Gegenstand der vorliegenden Erfindung ist ferner ein Verfahren zur Visualisierung von Stoffwechselvorgängen mit folgenden Schritten:
(1) Bereitstellung einer [¹⁸F]fluormarkierten aromatischen L-Aminosäure;
(2) Einbringen der Aminosäure aus Schritt (1) in einen lebenden Organismus, und
(3) Detektion der eingebrachten Aminosäure im lebenden Organismus,
wobei Schritt (1) mittels des vorstehend ausgeführten erfindungsgemäßen Verfahrens erfolgt. Dabei ist es bevorzugt, wenn Schritt (3) mittels eines Strahlungsdetektors, vorzugsweise eines Positron-Emissions-Tomographie-Scanners und/oder mit Hilfe der Autoradiographie erfolgt.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die vorliegende Erfindung wird nun an Hand von rein beispielhaften und nicht einschränkenden Ausführungsbeispielen näher erläutert.

### Beispiel 1: Herstellung der L-enantiomeren Markierungsprecursors

Die Herstellung der Markierungsprecursors werden im Anschluss mittels Fließdiagrammen skizziert. Als Substituenten wurde für Z=CHO und für Y=NO₂ gewählt.
A) [¹⁸F]-Phenylalanin-Precursor 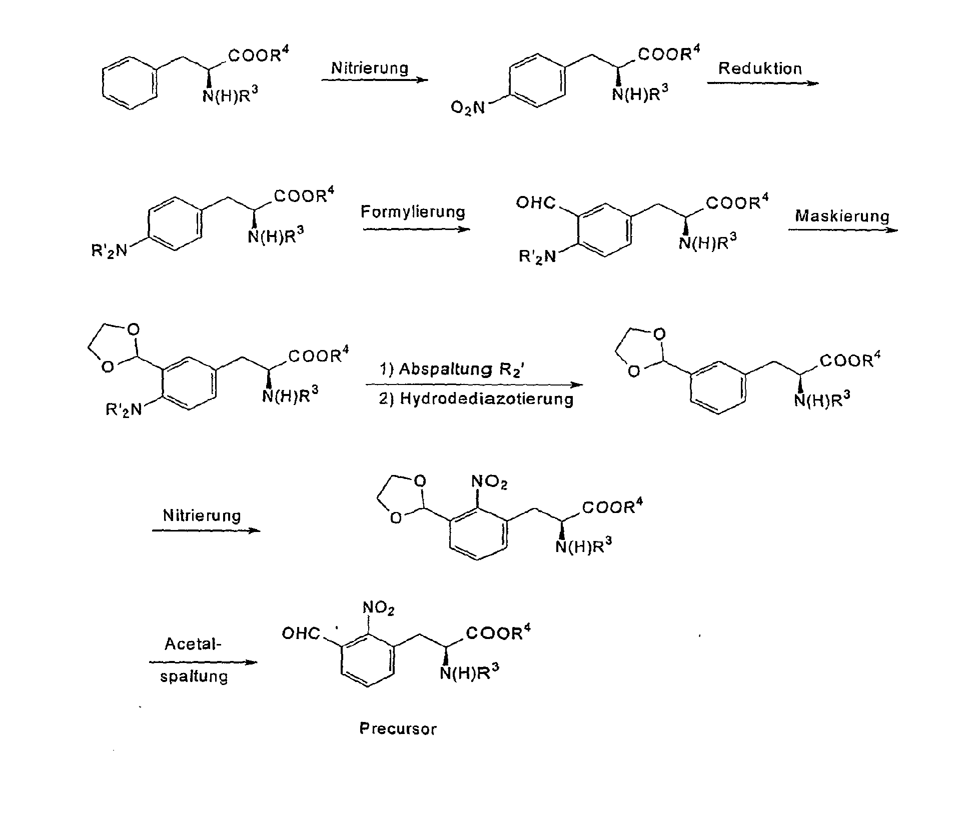
B) [¹⁸F]-Tyrosin-Precursor 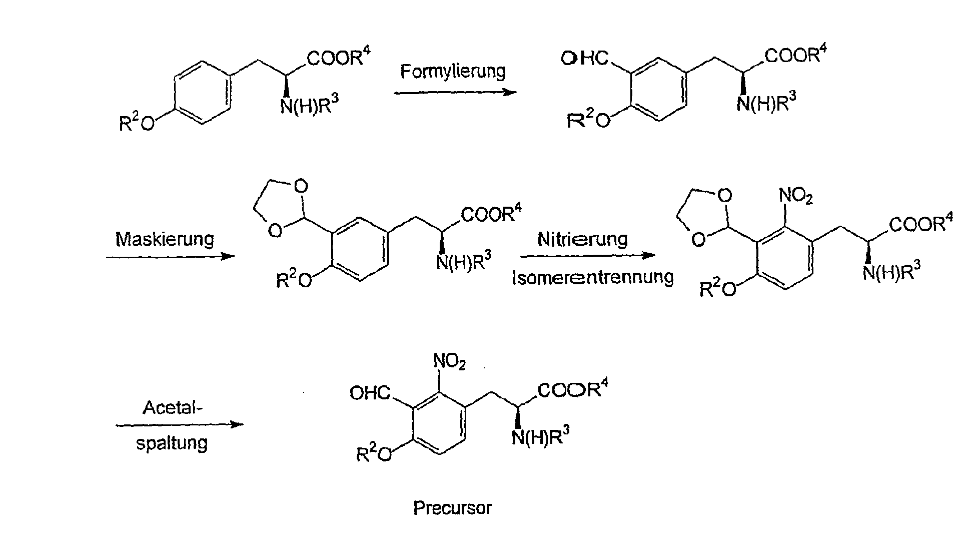
C) [¹⁸F]-5-hydroxy-Phenylalanin-Precursor 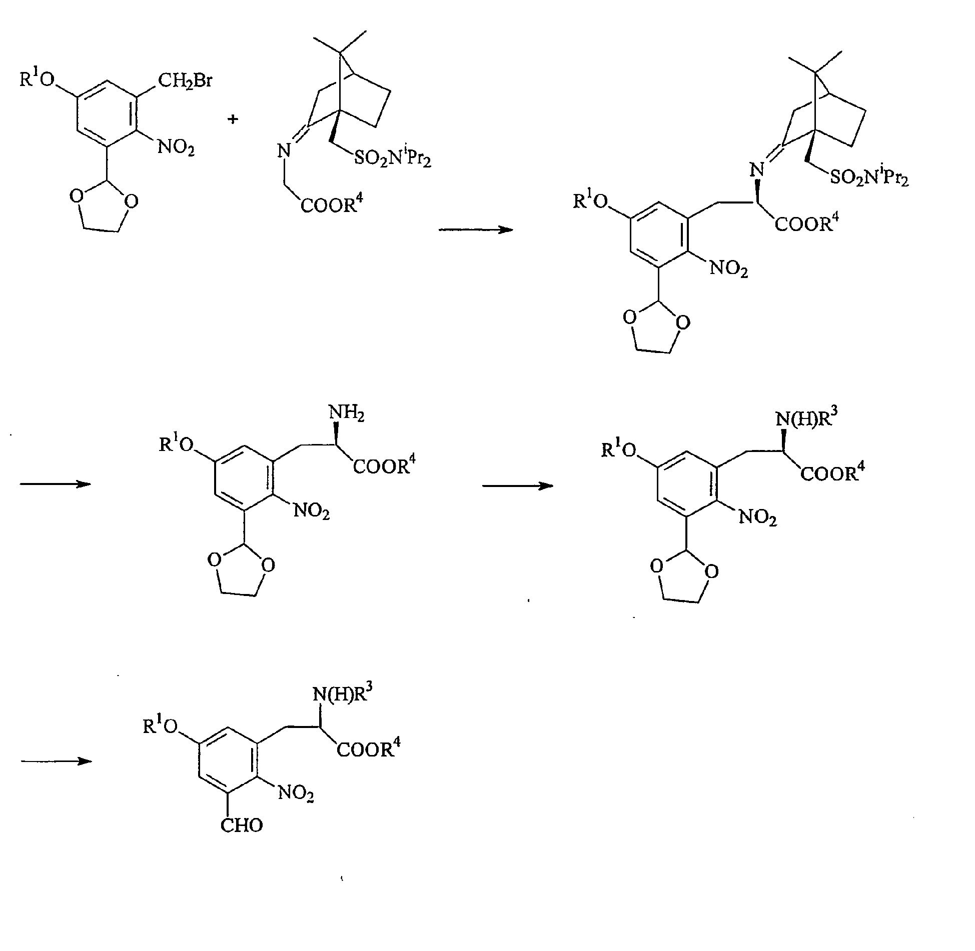
D) [¹⁸F]-DOPA-Precursor

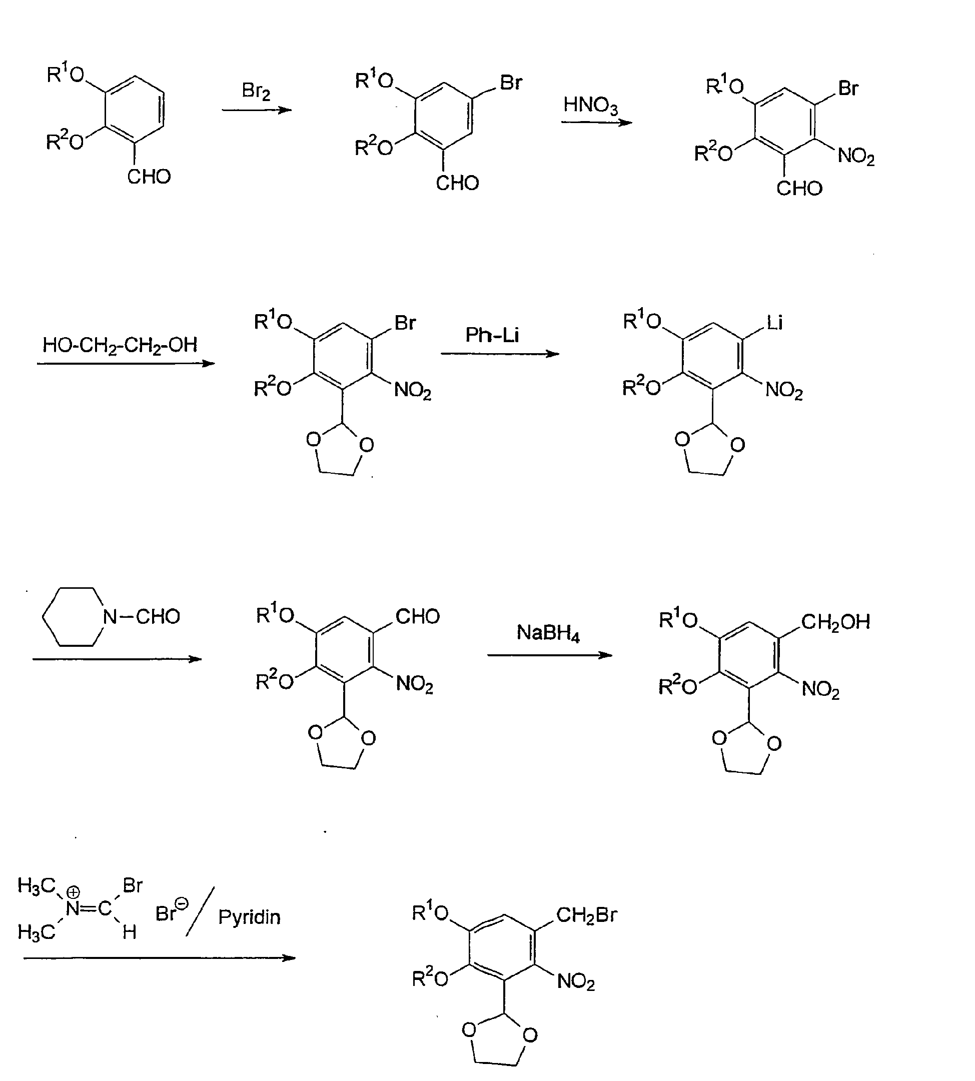

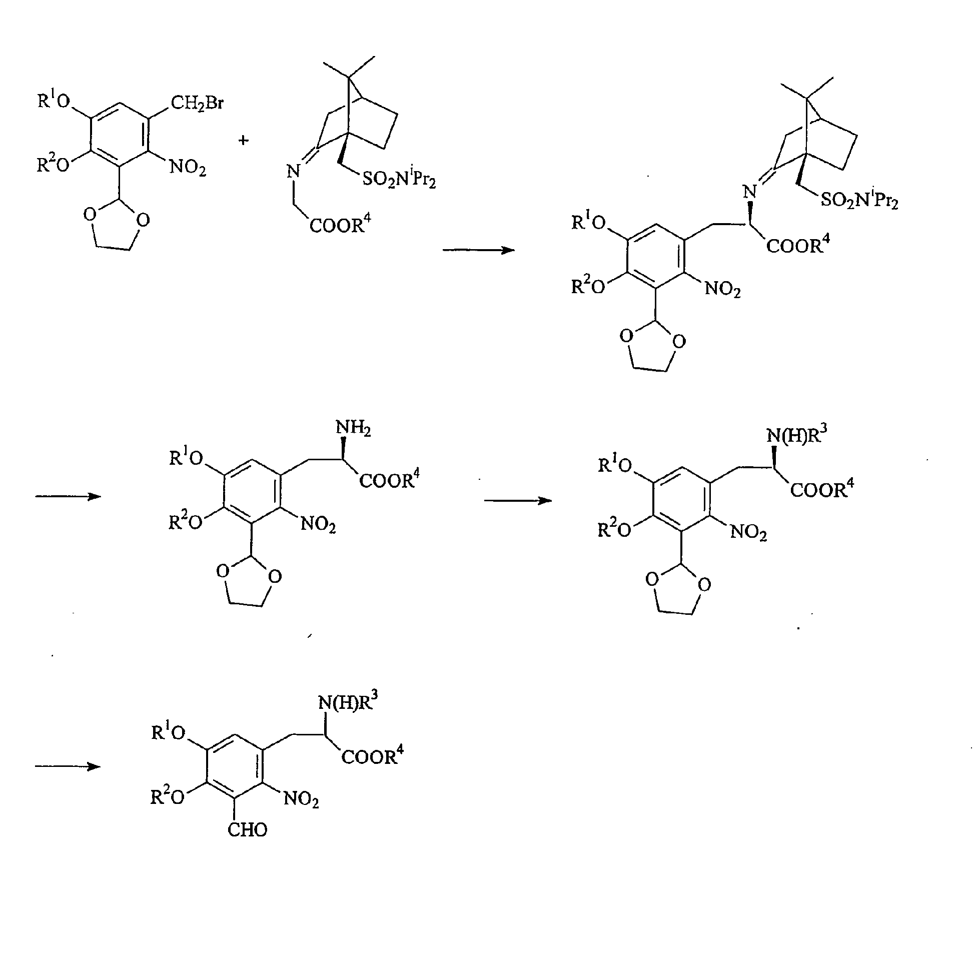

### Beispiel 2: Substitution der Abgangsgruppe Y durch das [¹⁸F]Fluorid-Ion

Im ersten Schritt der eigentlichen Synthesereaktion wird der Substituent Y, d.h. die Abgangsgruppe, gemäß im Stand der Technik allgemein bekannter Methoden durch ein [¹⁸F]Fluorid-Ion substituiert:

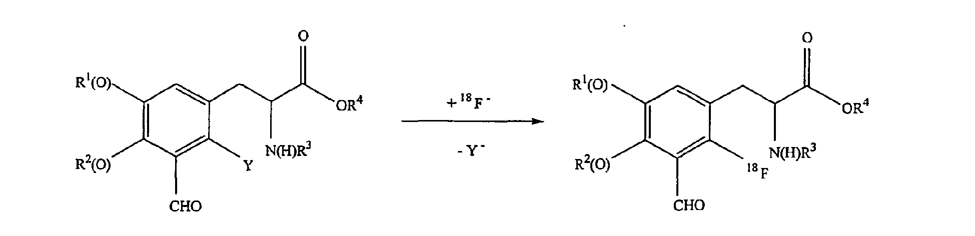

Auf Grund der hohen Elektronendichte im Benzolring, die bei der Herstellung von markiertem L-Tyrosin (R¹(O)=H, R²(O)=OH) und L-DOPA (R¹(O)=R²(O)=OH) auf Grund der Hydroxyl-Gruppen zusätzlich erhöht ist, dient dabei die stark elektronenziehende Gruppe CHO (aktivierende Gruppe) in direkter Nachbarschaft zur Abgangsgruppe Y der Erleichterung dieser Substitution.

### Beispiel 3: Abspaltung der aktivierenden Gruppe CHO

Im nächsten Schritt erfolgt die Abspaltung der aktivierenden Aldehydgruppe. Dies lässt sich zielgerichtet auf einfache Art und Weise mittels eines Decarbonylierungskatalysators (Tris-(triphenylphosphin)-rhodium(I)-chlorid und/oder Wilkinson-Katalysator) verwirklichen:

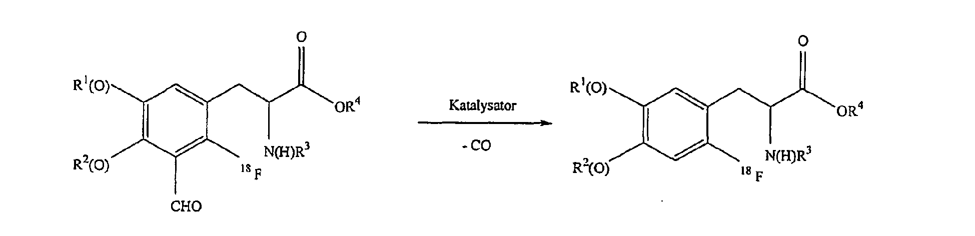

### Beispiel 4: Hydrolytische Abspaltung der Schutzgruppen R³, R⁴

Im nächsten Schritt erfolgt die Abspaltung der Schutzgruppen R³ und R⁴ durch Hydrolyse gemäß im Stand der Technik allgemein bekannter Methoden:

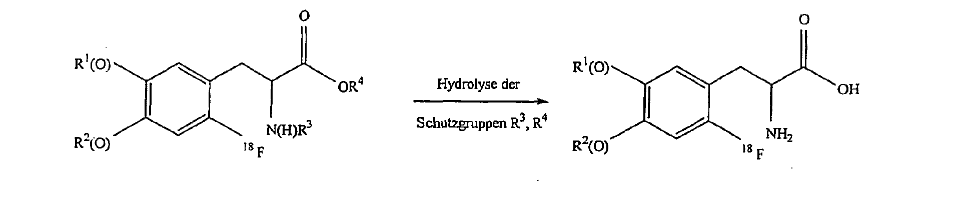

### Beispiel 5: Hydrolytische Abspaltung von R¹ und R²

Für die Herstellung von 2-[¹⁸F]Fluoro-L-phenylalanin ist dieser Schritt nicht erforderlich, da R¹(O)=R²(O)=H entsprechen. Sofern R¹(O) und R²(O) keinem Wasserstoffatom entsprechen, erfolgt in diesem letzten Syntheseschritt, der gleichzeitig mit der vorherigen Abspaltung von R³ und R⁴ erfolgt, die Abspaltung dieser Schutzgruppen R¹(O) und R²(O), verbunden mit einer Überführung in OH-Gruppen, d.h. zur Herstellung von 2-[¹⁸F]Fluoro-L-tyrosin wird R²(O) in R⁶=OH überführt, wobei R¹(O)=R⁵=H entspricht. Im Falle der Herstellung von 2-[¹⁸F]Fluoro-5-hydroxy-L-phenylalanin entspricht R²(O)=R⁶=H und R¹ wird in R⁵=OH überführt. Zur Herstellung von 6-[¹⁸F]Fluoro-3,4-dihydroxy-L-phenylalanin (FDOPA) wird R¹(O) in R⁵=OH und R²(O) in R⁶=OH überführt:

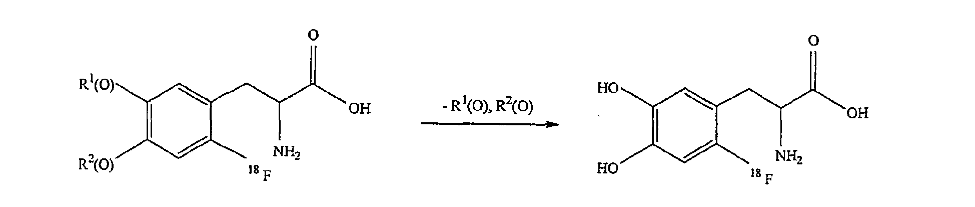

Die einzelnen Reaktionen sind im Stand der Technik, bezogen auf andere Verbindungen, grundsätzlich bekannt.

## Patentansprüche

1. Verfahren zur Herstellung [¹⁸F]fluormarkierter aromatischer L-Aminosäuren mit folgenden Schritten:
(1) Bereitstellung der nachfolgend dargestellten L-enantiomeren Verbindung in einem geeigneten Reaktionsmedium: 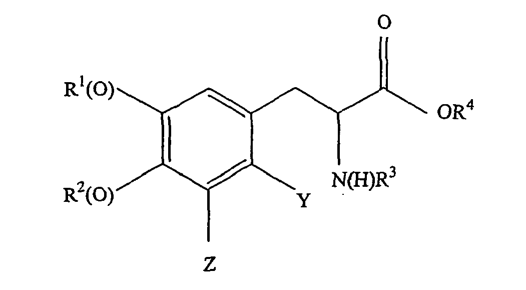 wobei R¹ und R² geeignete Schutzgruppen für OH-Gruppen sind, sofern eine Hydroxyaminosäure hergestellt werden soll;
wobei Z eine elektronenziehende Gruppe ist;
wobei Y eine Abgangsgruppe für eine nucleophile Substitution ist;
wobei R³ einer oder mehreren geeigneten Schutzgruppen für eine Aminofunktion entspricht;
wobei R⁴ einer geeigneten Schutzgruppe für eine Carboxylfunktion entspricht;
(2) nucleophile Substitution von Y durch ein negativ geladenes [¹⁸F]Fluorid-Ion zur Herstellung der nachfolgend dargestellten Verbindung: 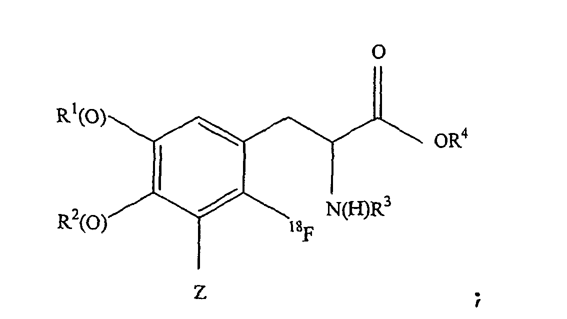
(3) Abspaltung von Z zur Herstellung der nachfolgend dargestellten Verbindung: 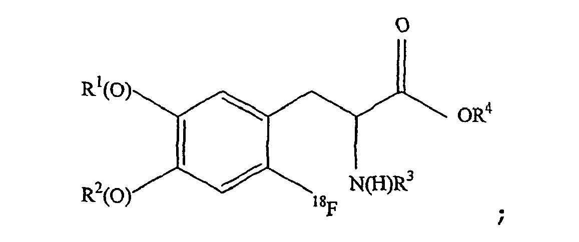
(4) hydrolytische Abspaltung von R³ und R⁴ zur Herstellung der nachfolgend dargestellten Verbindung: 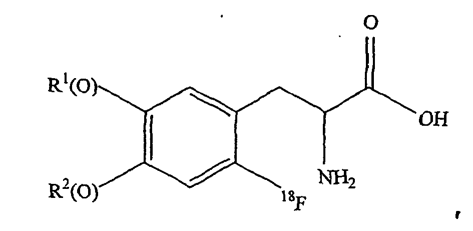 und
(5) hydrolytische Abspaltung von R¹ und R² zur Herstellung der nachfolgend dargestellten [¹⁸F]fluormarkierten aromatischen L-Aminosäure: 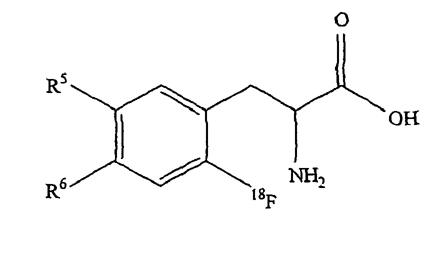 wobei R⁵ und R⁶ Substituenten sind, die jeweils einem H-Atom oder einer OH-Gruppe entsprechen.

2. Verfahren nach Anspruch 1, **dadurch gekennzeichnet, dass** R¹ und R² jeweils ausgewählt sind aus der Gruppe bestehend aus: CH₃, CH₂OCH₃, CH₂OCH₂(C₆H₅) , CH₂SCH₃, CH₂SCH₂(C₆H₅), CH₂OCH₂Cl, CH₂OCH₂Br, CH₂COC₆H₃-3,4-Cl, CH₂COC₆H₃-2,6-Cl₂, CH₂=CH₂, CH(CH₃)₂, c-C₆H₁₁, C(CH₃)₃, CH₂C₆H₅, 2,6-(CH₃)₂C₆H₃CH₂, 4-CH₃OCH₄CH₂, *o-*NO₂-C₆H₄CH₂, (CH₃)₂NCOC₆H₄CH₂, COCH₃, COC₆H₅, CO₂CH₃, COOCH₂CCl₃, CONHPh (Ph=Phenyl) und CONH*-i*-Bu (Bu=Butyl).

3. Verfahren nach Anspruch 1 oder 2, **dadurch gekennzeichnet, dass** R¹ und R² einem cyclischen Acetal entsprechen, das vorzugsweise ausgewählt ist aus der Gruppe bestehend aus: Methylen-, Dimethylmethylen-, Cyclohexyliden-, Diphenylmethylen-, Ethoxymethylenacetal und cyclischem Borsäureester.

4. Verfahren nach einem der vorherigen Ansprüche, **dadurch gekennzeichnet, dass** Z einem Substituenten zweiter Ordnung entspricht, der vorzugsweise ausgewählt ist aus der Gruppe bestehend aus: CHO, NO₂, SO₂Me (Me=Methyl) , NR₃⁺ (R=Alkylgruppe), CF₃, CN, COR (R=Alkyl/Aryl), COOH, Br, Cl und I.

5. Verfahren nach einem der vorherigen Ansprüche, **dadurch gekennzeichnet, dass** Y einem Substituenten entspricht, der ausgewählt ist aus der Gruppe bestehend aus: F, NO₂, OTs, Cl, Br, I, N₃ und NR₃⁺ (R=Alkyl/Aryl).

6. Verfahren nach einem der vorherigen Ansprüche, **dadurch gekennzeichnet, dass** R³ einem oder mehreren Substituenten entspricht, die ausgewählt sind aus der Gruppe bestehend aus: 9-Fluorenylmethylcarbamat, CO₂CH₂CCl₃, CO₂CH₂CH₂Ph (Ph=Phenyl) , CO₂C(CH₃)CHBr₂, CO₂C(CH₃)₂CCl₃, CO₂C(CH₃)₃, N-Hydroxypiperidinylcarbamat, CO₂CH₂Ph (Ph=Phenyl), CO₂CH₂-*p-*CH₃OC₆H₄, CO₂CH₂*-p*-NO₂C₆H₄, CHO; COCH₃, COCH₂Cl, COCCl₃, COCF₃, COC₆H₅, Phthalimid, Dithiasuccinimid, N-5-Dibenzosuberylamin, N-1,1-Dimethylthiomethylen, N-Benzyliden, N-1,3-Dithiolan-2-yliden, N-Diphenylmethylen, =CHN(CH₃)₂ und =CN(CH₂C₆H₅)₂.

7. Verfahren nach einem der vorherigen Ansprüche, **dadurch gekennzeichnet, dass** R⁴ einem oder mehreren Substituenten entspricht, die ausgewählt sind aus der Gruppe bestehend aus: CH₃, C₂H₅, CH₂OCH₃, CH₂SCH₃, CH₂OCH₂C₆H₅, CH₂CCl₃, C(CH₃)₃, CH₂C₆H₅, CH₂C₆H₂-2,4,6-(CH₃)₃.

8. Verfahren nach einem der vorherigen Ansprüche, **dadurch gekennzeichnet, dass** Schritt (3) mit Hilfe eines Decarbonylierungskatalysators, vorzugsweise mit Tris(triphenylphosphin)-rhodium(I)-chlorid und/oder konzentrierter Schwefelsäure (H₂SO_{4konz.}) und/oder Palladium auf Aktivkohle (Pd/C) und/oder einem Wilkinson-Katalysator, erfolgt, sofern Z CHO entspricht.

9. Verfahren nach einem der vorherigen Ansprüche, **dadurch gekennzeichnet, dass** sämtliche Schritte in einem einzigen Reaktionsgefäß durchgeführt werden.

10. Verfahren nach einem der vorherigen Ansprüche, **dadurch gekennzeichnet, dass** sämtliche Schritte automatisiert, vorzugsweise mit Hilfe eines Syntheseroboters, weiter bevorzugt mit Hilfe eines kompakten Syntheseautomaten, erfolgen.

11. Verfahren nach einem der vorherigen Ansprüche, **dadurch gekennzeichnet, dass** ein weiterer folgender Schritt durchgeführt wird:
(6) Herstellung eines Tracers durch Formulierung der [¹⁸F]fluormarkierten aromatischen L-Aminosäure mit einem pharmazeutisch akzeptablen Träger und/oder Lösungsmittel.

12. Verfahren zur Herstellung eines Diagnostikums mit folgenden Schritten:
(1) Bereitstellung einer [¹⁸F]fluormarkierten aromatischen L-Aminosäure, und
(2) Formulierung der Aminosäure aus Schritt (1) mit einem pharmazeutisch akzeptablen Träger und/oder Lösungsmittel sowie ggf. weiterer pharmazeutischer Hilfsstoffe,
**dadurch gekennzeichnet, dass** Schritt (1) mittels des Verfahrens nach einem der Ansprüche 1 bis 10 erfolgt.

13. Verfahren nach Anspruch 12, **dadurch gekennzeichnet, dass** das Diagnostikum zur Verwendung im Rahmen der Positron-Emissions-Tomographie (PET) vorgesehen ist.

14. Verfahren zur Visualisierung von Stoffwechselvorgängen mit folgenden Schritten:
(1) Bereitstellung einer [¹⁸F]fluormarkierten aromatischen L-Aminosäure;
(2) Einbringen der Aminosäure aus Schritt (1) in einen lebenden Organismus, und
(3) Detektion der eingebrachten Aminosäure im lebenden Organismus,
**dadurch gekennzeichnet, dass** Schritt (1) mittels des Verfahrens nach einem der Ansprüche 1 bis 13 erfolgt.

15. Verfahren nach Anspruch 14, **dadurch gekennzeichnet, dass** Schritt (3) mittels eines Strahlungsdetektors, vorzugsweise eines Positron-Emissions-Tomographie-Scanners und/oder mit Hilfe der Autoradiographie erfolgt.

16. Verwendung der nachfolgend dargestellten L-enantiomeren Verbindung: 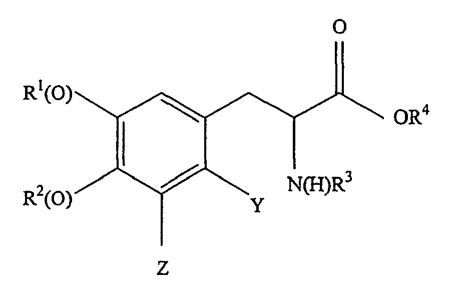 zur Herstellung [¹⁸F]fluormarkierter aromatischer L-Aminosäuren,
wobei R¹ und R² geeignete Schutzgruppen für OH-Gruppen sind;
wobei Z eine elektronenziehende Gruppe ist;
wobei Y eine Abgangsgruppe für eine nucleophile Substitution ist;
wobei R³ einer oder mehreren geeigneten Schutzgruppen für eine Aminofunktion entspricht;
wobei R⁴ einer geeigneten Schutzgruppe für eine Carboxylfunktion entspricht.

17. Verwendung nach Anspruch 16, **dadurch gekennzeichnet, dass** R¹ und R² jeweils ausgewählt sind aus der Gruppe bestehend aus: H, CH₃, CH₂OCH₃, CH₂OCH₂(C₆H₅), CH₂SCH₃, CH₂SCH₂(C₆H₅), CH₂OCH₂Cl, CH₂OCH₂Br, CH₂COC₆H₃-3,4-Cl, CH₂COC₆H₃-2,6-Cl₂, CH₂=CH₂, CH(CH₃)₂, C-C₆H₁₁, C(CH₃)₃, CH₂C₆H₅, 2,6-(CH₃)₂C₆H₃CH₂, 4-CH₃OCH₄CH₂, *o-*NO₂-C₆H₄CH₂, (CH₃)₂NCOC₆H₄CH₂, COCH₃, COC₆H₅, CO₂CH₃, COOCH₂CCl₃, CONHPh (Ph=Phenyl) und CONH*-i*-Bu (Bu=Butyl).

18. Verwendung nach Anspruch 16 oder Anspruch 17, **dadurch gekennzeichnet, dass** R¹ und R² einem cyclischen Acetal entsprechen, das vorzugsweise ausgewählt ist aus der Gruppe bestehend aus: Methylen-, Dimethylmethylen-, Cyclohexyliden-, Diphenylmethylen-, Ethoxymethylenacetal und cyclischem Borsäureester.

19. Verwendung nach einem der Ansprüche 16 bis 18, **dadurch gekennzeichnet, dass** Z einem Substituenten zweiter Ordnung entspricht, der vorzugsweise ausgewählt ist aus der Gruppe bestehend aus: CHO, NO₂, SO₂Me (Me=Methyl), NR₃⁺ (R=Alkylgruppe), CF₃, CN, COR (R=Alkyl/Aryl), COOH, Br, Cl und I.

20. Verwendung nach einem der Ansprüche 16 bis 19, **dadurch gekennzeichnet, dass** Y einem Substituenten entspricht, der ausgewählt ist aus der Gruppe bestehend aus: F, NO₂, OTs, Cl, Br, I, N₃ und NR₃⁺ (R=Alkyl/Aryl).

21. Verwendung nach einem der Ansprüche 16 bis 20, **dadurch gekennzeichnet, dass** R³ einem oder mehreren Substituenten entspricht, die ausgewählt sind aus der Gruppe bestehend aus: 9-Fluorenylmethylcarbamat, CO₂CH₂CCl₃, CO₂CH₂CH₂Ph (Ph=Phenyl), CO₂C(CH₃)CHBr₂, CO₂C(CH₃)₂CCl₃, CO₂C(CH₃)₃, N-Hydroxypiperidinylcarbamat, CO₂CH₂Ph (Ph=Phenyl), CO₂CH₂*-p-*CH₃OC₆H₄, CO₂CH₂*-p*-NO₂C₆H₄, CHO; COCH₃, COCH₂Cl, COCCl₃, COCF₃, COC₆H₅, Phthalimid, Dithiasuccinimid, N-5-Dibenzosuberylamin, N-1,1-Dimethylthiomethylen, N-Benzyliden, N-1,3-Dithiolan-2-yliden, N-Diphenylmethylen, =CHN(CH₃)₂ und =CN(CH₂C₆H₅)₂.

22. Verwendung nach einem der Ansprüche 16 bis 21, **dadurch gekennzeichnet, dass** R⁴ einem oder mehreren Substituenten entspricht, die ausgewählt sind aus der Gruppe bestehend aus: CH₃, C₂H₅, CH₂OCH₃, CH₂SCH₃, CH₂OCH₂C₆H₅, CH₂CCl₃, C(CH₃)₃, CH₂C₆H₅, CH₂C₆H₂-2,4,6-(CH₃)₃.

23. L-enantiomere Verbindung, **gekennzeichnet durch** nachstehend wiedergegebene Strukturformel: 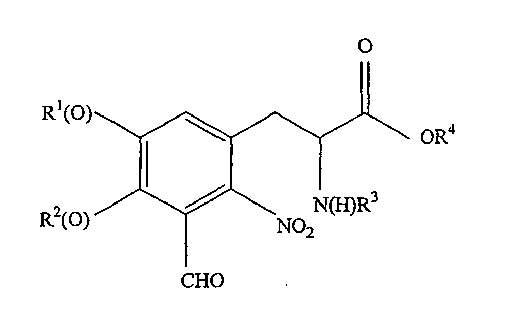 wobei R¹ und R² geeignete Schutzgruppen für OH-Gruppen sind;
wobei R³ eine geeignete Schutzgruppe für eine Aminofunktion ist;
wobei R⁴ eine geeignete Schutzgruppe für eine Carboxylfunktion ist;

24. L-enantiömere Verbindung nach Anspruch 23, **dadurch gekennzeichnet, dass** R¹ und R² jeweils ausgewählt sind aus der Gruppe bestehend aus: H, CH₃, CH₂OCH₃, CH₂OCH₂(C₆H₅), CH₂SCH₃, CH₂SCH₂(C₆H₅), CH₂OCH₂Cl, CH₂OCH₂Br, CH₂COC₆H₃-3,4-Cl, CH₂COC₆H₃-2,6-Cl₂, CH₂=CH₂, CH(CH₃)₂, c-C₆H₁₁, C(CH₃)₃, CH₂C₆H₅, 2,6-(CH₃)₂C₆H₃CH₂, 4-CH₃OCH₄CH₂, O-NO₂-C₆H₄CH₂, (CH₃) ₂NCOC₆H₄CH₂, COCH₃, COC₆H₅, CO₂CH₃, COOCH₂CCl₃, CONHPh (Ph=Phenyl) und CONH*-i*-Bu (Bu=Butyl).

25. L-enantiomere Verbindung nach Anspruch 23, **dadurch gekennzeichnet, dass** R¹ und R² einem cyclischen Acetal entsprechen, das vorzugsweise ausgewählt ist aus der Gruppe bestehend aus: Methylen-, Dimethylmethylen-, Cyclohexyliden-, Diphenylmethylen-, Ethoxymethylenacetal und cyclischem Borsäureester.

26. L-enatiomere Verbindung nach einem der vorherigen Ansprüche, **dadurch gekennzeichnet, dass** R³ einem oder mehreren Substituenten entspricht, die ausgewählt sind aus der Gruppe bestehend aus: 9-Fluorenylmethylcarbamat, CO₂CH₂CCl₃, CO₂CH₂CH₂Ph (Ph=Phenyl), CO₂C(CH₃)CHBr₂, CO₂C(CH₃)₂CCl₃, CO₂C(CH₃)₃, N-Hydroxypiperidinylcarbamat, CO₂CH₂Ph (Ph=Phenyl), CO₂CH₂*-p*-CH₃OC₆H₄, CO₂CH₂*-p*-NO₂C₆H₄, CHO; COCH₃, COCH₂Cl, COCCl₃, COCF₃, COC₆H₅, Phthalimid, Dithiasuccinimid, N-5-Dibenzosuberylamin; N-1,1-Dimethylthiomethylen, N-Benzyliden, N-1,3-Dithiolan-2-yliden, N-Diphenylmethylen, =CHN(CH₃)₂ und =CN(CH₂C₆H₅)₂.

27. L-enatiomere Verbindung nach einem der vorherigen Ansprüche, **dadurch gekennzeichnet, dass** R⁴ einem oder mehreren Substituenten entspricht, die ausgewählt sind aus der Gruppe bestehend aus: CH₃, C₂H₅, CH₂OCH₃, CH₂SCH₃, CH₂OCH₂C₆H₅, CH₂CCl₃, C(CH₃)₃, CH₂C₆H₅, CH₂C₆H₂-2,4,6-(CH₃)₃.

## Claims

1. A method for preparing [¹⁸F]fluorine labeled aromatic L-amino acids comprising the following steps:
(1) providing the following L-enantiomeric compound in an appropriate reaction medium: 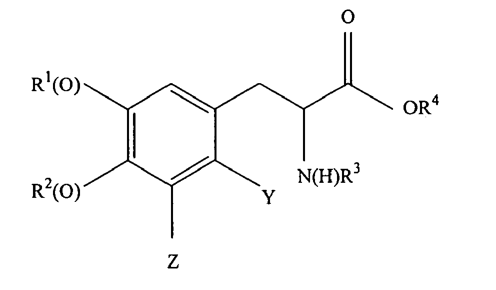 whereby R¹ and R² are appropriate protecting groups for OH groups, provided a hydroxy amino acid is to be prepared;
whereby Z is an electron-attracting group;
whereby Y is a leaving group for a nucleophilic substitution;
whereby R³ corresponds to one or several appropriate protecting group(s) for an amino function;
whereby R⁴ corresponds to an appropriate protecting group for a carboxyl function;
(2) nucleophilic substitution of Y for a negatively charged [¹⁸F]fluorine ion to prepare the following compound: 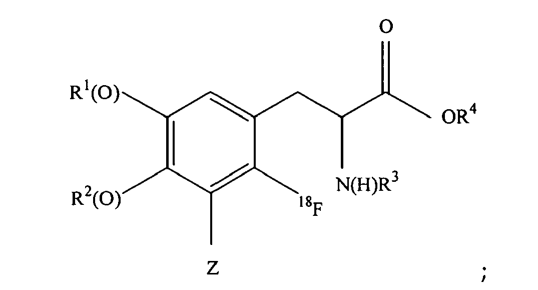
(3) cleaving-off of Z to prepare the following compound: 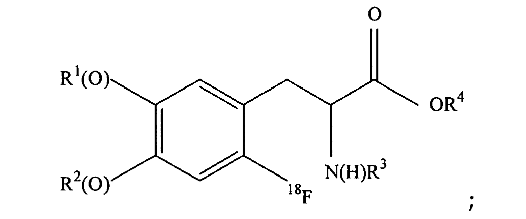
(4) hydrolytic cleaving-off of R³ and R⁴ to prepare the following compound: 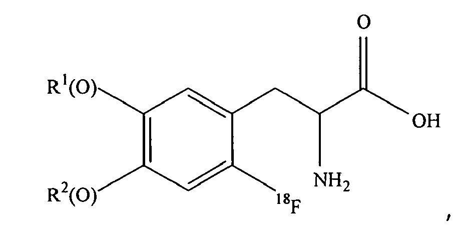 and
(5) hydrolytic cleaving-off of R¹ and R² to prepare the following [¹⁸F]fluorine labeled aromatic L-amino acid: 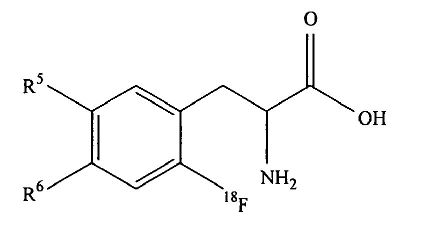 whereby R⁵ and R⁶ are substituents, which are in each case an H atom or an OH group.

2. Method according to claim 1, **characterized in that** R¹ and R² are each selected from the group consisting of: CH₃, CH₂OCH₃, CH₂OCH₂(C₆H₅), CH₂SCH₃, CH₂SCH₂(C₆H₅), CH₂OCH₂Cl, CH₂OCH₂Br, CH₂COC₆H₃-3,4-Cl, CH₂COC₆H₃-2,6-Cl₂, CH₂=CH₂, CH(CH₃)₂, c-C₆H₁₁, C(CH₃)₃, CH₂C₆H₅, 2,6-(CH₃)₂C₆H₃CH₂, 4-CH₃OCH₄CH₂, *o-*NO₂-C₆H₄CH₂, (CH₃)₂NCOC₆H₄CH₂, COCH₃, COC₆H₅, CO₂CH₃, COOCH₂CCl₃, CONHPh (Ph=phenyl), and CONH*-i*-Bu (Bu=butyl).

3. Method according to claim 1 or 2, **characterized in that** R¹ and R² correspond to a cyclic acetal which is preferably selected from the group consisting of: methylene, dimethylmethylene, cyclohexylidene, diphenylmethylene, ethoxymethylenacetal, and cyclic boric acid ester.

4. Method according to any of the preceding claims, **characterized in that** Z is a substituent of second order which is preferably selected from the group consisting of : CHO, NO₂, SO₂Me (Me=methyl), NR₃⁺ (R=alkyl group), CF₃, CN, COR (R=alkyl/aryl), COOH, Br, Cl, and I.

5. Method according to any of the preceding claims, **characterized in that** Y is a substituent which is selected from the group consisting of: F, NO₂, OTs, Cl, Br, I, N₃, and NR₃⁺ (R=alkyl/aryl).

6. Method according to any of the preceding claims, **characterized in that** R³ corresponds to one or several substituent(s) which is/are selected from the group consisting of: 9-fluorenylmethylcarbamate, CO₂CH₂CCl₃, CO₂CH₂CH₂Ph (Ph=phenyl), CO₂C(CH₃)CHBr₂, CO₂C(CH₃)₂CCl₃, CO₂C(CH₃)₃, N-hydroxypiperidinylcarbamate, CO₂CH₂Ph (Ph=phenyl), CO₂CH₂*-p-*CH₃OC₆H₄, CO₂CH₂*-p*-NO₂C₆H₄, CHO; COCH₃, COCH₂Cl, COCCl₃, COCF₃, COC₆H₅, phthalimide, dithiasuccinimide, N-5-dibenzosuberylamine, N-1,1-dimethylthiomethylene, N-benzylidene, N-1,3-dithiolan-2-ylidene, N-diphenylmethylene, =CHN(CH₃)₂, and =CN(CH₂C₆H₅)₂.

7. Method according to any of the preceding claims, **characterized in that** R⁴ corresponds to one or several substituent(s) which is/are selected from the group consisting of: CH₃, C₂H₅, CH₂OCH₃, CH₂SCH₃, CH₂OCH₂C₆H₅, CH₂CCl₃, C(CH₃)₃, CH₂C₆H₅, CH₂C₆H₂-2,4,6-(CH₃)₃.

8. Method according to any of the preceding claims, **characterized in that** step (3) is performed by the aid of a decarbonylization catalyser, preferably of a Tris(triphenylphosphine)-rhodium(I)-chloride and/or concentrated sulphoric acid (H₂SO_{4conc}.) and/or palladium on activated carbon (Pd/C) and/or a Wilkinson catalyser (rhodium catalyser)

9. Method according to any of the proceeding claims, **characterized in that** all steps are performed in a single reaction vial.

10. Method according to any of the preceding claims, **characterized in that** all steps are performed automatized, preferably by the aid of a synthesis robot, further preferred by the aid of a compact synthesis apparatus.

11. Method according to any of the preceding claims, **characterized in that** the following further step is performed:
(6) Preparation of a tracer by formulating the [¹⁸F]fluorine labeled aromatic L-amino acid with a pharmaceutically acceptable carrier and/or a solvent.

12. Method for preparing a diagnostic agent comprising the following steps:
(1) providing a [¹⁸F]fluorine labeled aromatic L-amino acid, and
(2) formulating the amino acid of step (1) with a pharmaceutically acceptable carrier and/or a solvent and, if applicable, further pharmaceutical excipients,
**characterized in that** step (1) is performed by means of the method according to any of the claims 1 to 10.

13. Method according to claim 12, **characterized in that** the diagnostic agent is designated for a use within the positron emission tomography (PET).

14. Method for visualizing metabolic processes comprising the following steps:
(1) providing a [¹⁸F]fluorine labeled aromatic L-amino acid;
(2) introducing the amino acid of step (1) into a living organism, and
(3) detection of the introduced amino acid in the living organism,
**characterized in that** step (1) is performed by the method according to any of the claims 1 to 13.

15. Method according to claim 14, **characterized in that** step (3) is performed by means of a radiation detector, preferably a positron emission tomography scanner and/or by the aid of autoradiography.

16. Use of the following L-enantiomeric compound: 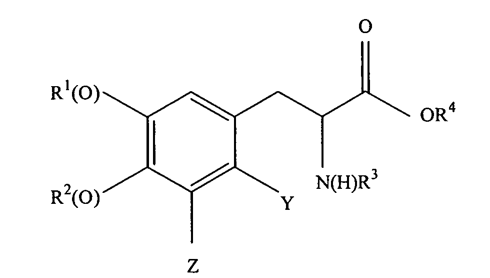 for preparing [¹⁸F]fluorine labeled aromatic L-amino acids,
whereby R¹ and R² are appropriate protecting groups for OH groups;
whereby Z is an electron-attracting group;
whereby Y is a leaving group for a nucleophilic substitution;
whereby R³ corresponds to one or several appropriate protecting group(s) for an amino function;
whereby R⁴ is an appropriate protecting group for a carboxyl function.

17. Use according to claim 16, **characterized in that** R¹ and R² are each selected from the group consisting of: H, CH₃, CH₂OCH₃, CH₂OCH₂(C₆H₅), CH₂SCH₃, CH₂SCH₂(C₆H₅), CH₂OCH₂Cl, CH₂OCH₂Br, CH₂COC₆H₃-3,4-Cl, CH₂COC₆H₃-2,6-Cl₂, CH₂=CH₂, CH(CH₃)₂, c-C₆H₁₁, C(CH₃)₃, CH₂C₆H₅, 2,6-(CH₃)₂C₆H₃CH₂, 4-CH₃OCH₄CH₂, *o-*NO₂-C₆H₄CH₂, (CH₃)₂NCOC₆H₄CH₂, COCH₃, COC₆H₅, CO₂CH₃, COOCH₂CCl₃, CONHPh (Ph=phenyl), and CONH*-i*-Bu (Bu=butyl).

18. Use according to claims 16 to 17, **characterized in that** R¹ and R² correspond to a cyclic acetal which is preferably selected from the group consisting of: methylene, dimethylmethylene, cyclohexylidene, diphenylmethylene, ethoxymethylenacetal, and cyclic boric acid ester.

19. Use according to claims 16 to 18, **characterized in that** Z corresponds to a substituent of second order which is preferably selected from the group consisting of : CHO, NO₂, SO₂Me (Me=methyl), NR₃⁺ (R=alkyl group), CF₃, CN, COR (R=alkyl/aryl), COOH, Br, Cl, and I.

20. Use according to claims 16 to 19, **characterized in that** Y corresponds to a substituent which is selected from the group consisting of: F, NO₂, OTs, Cl, Br, I, N₃, and NR₃⁺ (R=alkyl/a-ryl).

21. Use according to claims 16 to 20, **characterized in that** R³ corresponds to one or several substituent(s) which is/are selected from the group consisting of: 9-fluorenylmethylcarbamate, CO₂CH₂CCl₃, CO₂CH₂CH₂Ph (Ph=phenyl), CO₂C(CH₃)CHBr₂, CO₂C(CH₃)₂CCl₃, CO₂C(CH₃)₃, N-hydroxypiperidinylcarbamate, CO₂CH₂Ph (Ph=phenyl), CO₂CH₂*-p*-CH₃OC₆H₄, CO₂CH₂*-p*-NO₂C₆H₄, CHO; COCH₃, COCH₂Cl, COCCl₃, COCF₃, COC₆H₅, phthalimide, dithiasuccinimide, N-5-dibenzosuberylamine, N-1,1-dimethylthiomethylene, N-benzylidene, N-1,3-dithiolan-2-ylidene, N-diphenylmethylene, =CHN(CH₃)₂, and =CN(CH₂C₆H₅)₂.

22. Use according to claim 16, **characterized in that** R⁴ corresponds to one or several substituent(s) which is/are selected from the group consisting of: CH₃, C₂H₅, CH₂OCH₃, CH₂SCH₃, CH₂OCH₂C₆H₅, CH₂CCl₃, C(CH₃)₃, CH₂C₆H₅, CH₂C₆H₂-2,4,6-(CH₃)₃.

23. L-enantiomeric compound, **characterized by** the following chemical structure: 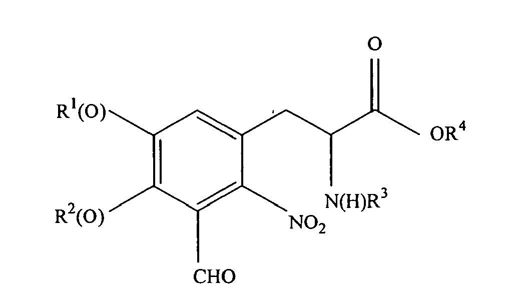 whereby R¹ and R² are appropriate protecting groups for OH groups;
whereby R³ is an appropriate protecting group for an amino function;
whereby R⁴ is an appropriate protecting group for a carboxyl function.

24. L-enantiomeric compound according to claim 23, **characterized in that** R¹ and R² are each selected from the group consisting of: H, CH₃, CH₂OCH₃, CH₂OCH₂(C₆H₅), CH₂SCH₃, CH₂SCH₂(C₆H₅), CH₂OCH₂Cl, CH₂OCH₂Br, CH₂COC₆H₃-3,4-Cl, CH₂COC₆H₃-2,6-Cl₂, CH₂=CH₂, CH(CH₃)₂, c-C₆H₁₁, C(CH₃)₃, CH₂C₆H₅, 2,6-(CH₃)₂C₆H₃CH₂, 4-CH₃OCH₄CH₂, *o-*NO₂-C₆H₄CH₂, (CH₃)₂NCOC₆H₄CH₂, COCH₃, COC₆H₅, CO₂CH₃, COOCH₂CCl₃, CONHPh (Ph=phenyl), and CONH*-i*-Bu (Bu=butyl).

25. L-enantiomeric compound according to claim 23 **characterized in that** R¹ and R² correspond to a cyclic acetal which is preferably selected from the group consisting of: methylene, dimethylmethylene, cyclohexylidene, diphenylmethylene, ethoxymethylenacetal, and cyclic boric acid ester.

26. L-enantiomeric compound according to any of the preceding claims, **characterized in that** R³ corresponds to one or several substituent(s) which is/are selected from the group consisting of: 9-fluorenylmethylcarbamate, CO₂CH₂CCl₃, CO₂CH₂CH₂Ph (Ph=phenyl), CO₂C(CH₃)CHBr₂, CO₂C(CH₃)₂CCl₃, CO₂C(CH₃)₃, N-hydroxypiperidinylcarbamate, CO₂CH₂Ph (Ph=phenyl), CO₂CH₂*-p*-CH₃OC₆H₄, CO₂CH₂*-p*-NO₂C₆H₄, CHO; COCH₃, COCH₂Cl, COCCl₃, COCF₃, COC₆H₅, phthalimide, dithiasuccinimide, N-5-dibenzosuberylamine, N-1,1-dimethylthiomethylene, N-benzylidene, N-1,3-dithiolan-2-ylidene, N-diphenylmethylene, =CHN(CH₃)₂, and =CN(CH₂C₆H₅)₂.

27. L-enantiomeric compound according to any of the proceeding claims, **characterized in that** R⁴ corresponds to one or several substituents which is/are selected from the group consisting of: CH₃, C₂H₅, CH₂OCH₃, CH₂SCH₃, CH₂OCH₂C₆H₅, CH₂CCl₃, C(CH₃)₃, CH₂C₆H₅, CH₂C₆H₂-2,4,6-(CH₃)₃.

## Revendications

1. Procédé de production de L-amino-acides aromatiques marqués par le fluor [¹⁸F] comportant les étapes suivantes :
(1) mise à disposition du composé L-énantiomère représenté ci-après, dans un milieu réactionnel approprié : 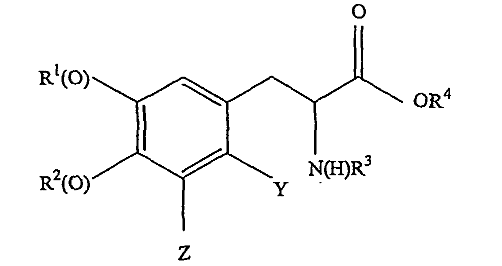 dans lequel R¹ et R² sont des groupes protecteurs appropriés des groupes OH, dans la mesure où un hydroxy-amino-acide doit être produit ;
Z est un groupe attracteur d'électron ;
Y est un groupe partant d'une substitution nucléophile ;
R³ correspond à un ou plusieurs groupes protecteurs appropriés pour une fonction amino ;
R⁴ correspond à un groupe protecteur approprié d'une fonction carboxyle ;
(2) substitution nucléophile de Y par un ion fluorure [¹⁸F] chargé négativement pour produire le composé représenté ci-après : 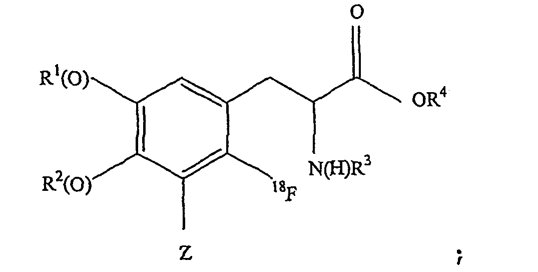
(3) clivage de Z pour produire le composé représenté ci-après : 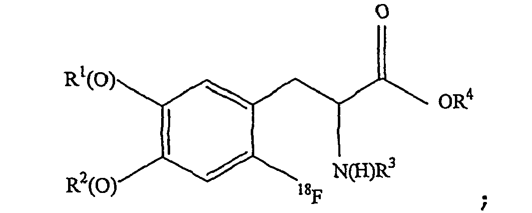
(4) clivage hydrolytique de R³ et R⁴ pour produire le composé représenté ci-après : 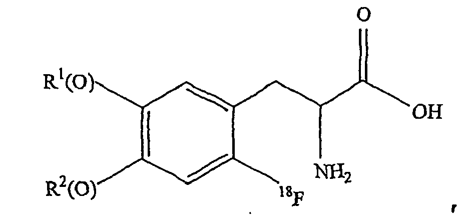 et
(5) clivage hydrolytique de R¹ et R² pour produire le L-amino-acide aromatique marqué par le fluor [¹⁸F] représenté ci-après : 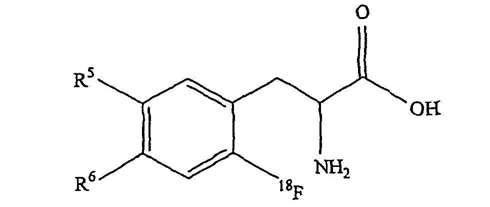 R⁵ et R⁶ étant des substituants qui correspondent respectivement à un atome de H ou à un groupe OH.

2. Procédé selon la revendication 1, **caractérisé en ce que** R¹ et R² sont choisis respectivement dans le groupe comprenant : CH₃, CH₂OCH₃, CH₂OCH₂(C₆H₅), CH₂SCH₃, CH₂SCH₂(C₆H₅), CH₂OCH₂Cl, CH₂OCH₂Br, CH₂COC₆H₃-3,4-Cl, CH₂COC₆H₃-2,6-Cl₂, CH₂=CH₂, CH(CH₃)₂, c-C₆H₁₁, C(CH₃)₃, CH₂C₆H₅, 2,6-(CH₃)₂C₆H₃CH₂, 4-CH₃OCH₄CH₂, *o-*NO₂-C₆H₄CH₂, (CH₃)₂NCOC₆H₄CH₂, COCH₃, COC₆H₅, CO₂CH₃, COOCH₂CCl₃, CONHPh (Ph=phényle) et CONH*-i*-Bu (Bu = butyle).

3. Procédé selon la revendication 1 ou 2, **caractérisé en ce que** R¹ et R² correspondent à un acétal cyclique qui est choisi de préférence dans le groupe comprenant : les méthylène-acétal, diméthylène-acétal, cyclohexylidène-acétal, diphénylméthylène-acétal, éthoxyméthylène-acétal et l'ester d'acide borique cyclique.

4. Procédé selon l'une des revendications précédentes, **caractérisé en ce que** Z correspond à un substituant de second ordre qui est choisi de préférence dans le groupe comprenant : CHO, NO₂, SO₂Me (Me = méthyle), NR3⁺ (R = un groupe alkyle), CF₃, CN, COR (R = alkyle/aryle), COOH, Br, CI et I .

5. Procédé selon l'une des revendications précédentes, **caractérisé en ce que** Y correspond à un substituant qui est choisi dans le groupe comprenant : F, NO₂, OTs, CI, Br, I, N₃ et NR₃⁺ (R=alkyle/aryle).

6. Procédé selon l'une des revendications précédentes, **caractérisé en ce que** R³ correspond à un ou plusieurs substituants qui sont choisis dans le groupe comprenant : le 9-fluorénylméthylcarbamate, CO₂CH₂CCl₃, CO₂CH₂CH₂Ph (Ph = phényle), CO₂C(CH₃)CHBr₂, CO₂C(CH₃ₕCCl₃, CO₂C(CH₃)₃, N-hydroxypipéridinylcarbamate, CO₂CH₂Ph (Ph = phényle), CO₂CH₂-O-CH₃OC₆H₄, CO₂CH₂-O-NO₂C₆H₄, CHO; COCH₃, COCH₂Cl, COCCl₃, COCF₃, COC₆H₅, phtalimide, dithiasuccinimide, N-5-dibenzosubérylamine, N-1,1-diméthylthiométhylène, N-benzylidène, N-1,3-dithiolan-2-ylidène, N-diphénylméthylène, =CHN(CH₃)₂ et =CN(CH₂C₆H₅)₂.

7. Procédé selon l'une des revendications précédentes, **caractérisé en ce que** R⁴ correspond à un ou plusieurs substituants qui sont choisis dans le groupe comprenant CH₃, C₂H₅, CH₂OCH₃, CH₂SCH₃, CH₂OCH₂C₆H₅, CH₂CCl₃, C(CH₃)₃, CH₂C₆H₅, CH₂C₆H₂-2,4,6-(CH₃)₃.

8. Procédé selon l'une des revendications précédentes, **caractérisé en ce que** l'étape (3) s'effectue à l'aide d'un catalyseur de décarbonylation, de préférence avec du chlorure de tris(triphénylphosphine)-rhodium(I) et/ou de l'acide sulfurique concentré (H₂SO_{4conc}.) et/ou de palladium sur charbon actif (Pd/C) et/ou d'un catalyseur de Wilkinson, dans la mesure où Z correspond à CHO.

9. Procédé selon l'une des revendications précédentes, **caractérisé en ce que** toutes les étapes sont réalisées dans un récipient réactionnel unique.

10. Procédé selon l'une des revendications précédentes, **caractérisé en ce que** toutes les étapes s'effectuent de manière automatisée, de préférence à l'aide d'un robot de synthèse, de manière davantage préférée, à l'aide d'un automate de synthèse compact.

11. Procédé selon l'une des revendications précédentes, **caractérisé en ce qu'**une autre étape est réalisée :
(6) production d'un traceur par formulation du L-amino-acide aromatique marqué par le fluor [¹⁸F] avec un support et/ou solvant pharmaceutiquement acceptable.

12. Procédé de production d'un diagnostic comportant les étapes suivantes :
(1) mise à disposition d'un L-amino-acide aromatique marqué par le fluor [¹⁸F], et
(2) formulation de l'acide aminé de l'étape (1) avec un support et/ou un solvant pharmaceutiquement acceptable et éventuellement, d'autres adjuvants pharmaceutiques,
**caractérisé en ce que** l'étape (1) s'effectue au moyen du procédé selon l'une des revendications 1 à 10.

13. Procédé selon la revendication 12, **caractérisé en ce que** le diagnostic est prévu pour l'utilisation dans le cadre de la tomographie par émission de positrons (TEP).

14. Procédé de visualisation de processus métaboliques comportant les étapes suivantes :
(1) mise à disposition d'un L-amino-acide aromatique marqué par le fluor [¹⁸F];
(2) intégration de l'acide aminé de l'étape (1) dans un organisme vivant, et
(3) détection de l'acide aminé intégré dans l'organisme vivant,
**caractérisé en ce que** l'étape (1) s'effectue au moyen du procédé selon l'une des revendications 1 à 13.

15. Procédé selon la revendication 14, **caractérisé en ce que** l'étape (3) s'effectue au moyen d'un détecteur de rayonnement, de préférence, un scanner de tomographie par émission de positron et/ou à l'aide de l'autoradiographie.

16. Utilisation du composé de L-énantiomères représenté ci-après : 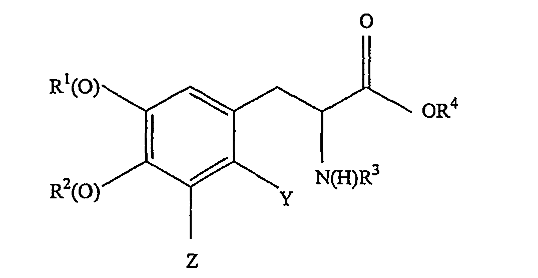 pour la production de L-amino-acides aromatiques marqués par le fluor [¹⁸F],
dans lequel R¹ et R² sont des groupes protecteurs appropriés des groupes OH ;
Z est un groupe attracteur d'électron ;
Y est un groupe partant d'une substitution nucléophile ;
R³ correspond à un ou plusieurs groupes protecteurs appropriés pour une fonction amino ;
R⁴ correspond à un groupe protecteur d'une fonction carboxyle.

17. Utilisation selon la revendication 16, **caractérisée en ce que** R¹ et R² sont choisis respectivement dans le groupe comprenant : H, CH₃, CH₂OCH₃, CH₂OCH₂(C₆H₅), CH₂SCH₃, CH₂SCH₂(C₆H₅), CH₂OCH₂Cl, CH₂OCH₂Br, CH₂COC₆H₃-3,4-Cl, CH₂COC₆H₃-2,6-Cl₂, CH₂=CH₂, CH(CH₃)₂, C-C₆H₁₁, C(CH₃)₃, CH₂C₆H₅, 2,6-(CH₃)₂C₆H₃CH₂, 4-CH₃OCH₄CH₂, o-NO₂-C₆H₄CH₂, (CH₃)₂NCOC₆H₄CH₂, COCH₃, COC₆H₅, CO₂CH₃, COOCH₂CCl₃, CONHPh (Ph=phényle) et CONH-i-Bu (Bu = butyle).

18. Utilisation selon la revendication 16 ou la revendication 17, **caractérisé en ce que** R¹ et R² correspondent à un acétal cyclique qui est choisi de préférence dans le groupe comprenant : les méthylène-acétal, diméthylène-acétal, cyclohexylidène-acétal, diphénylméthylène-acétal, éthoxyméthylène-acétal et un ester d'acide borique cyclique.

19. Utilisation selon l'une des revendications 16 à 18, **caractérisé en ce que** Z correspond à un substituant de second ordre qui est choisi de préférence dans le groupe comprenant : CHO, NO₂, SO₂Me (Me = méthyle), NR3⁺ (R = un groupe alkyle), CF₃, CN, COR (R = alkyle/aryle), COOH, Br, CI et I.

20. Utilisation selon l'une des revendications 16 à 19, **caractérisée en ce que** Y correspond à un substituant qui est choisi dans le groupe comprenant : F, NO₂, OTs, CI, Br, I, N₃ et NR₃⁺ (R=alkyle/aryle).

21. Utilisation selon l'une des revendications 16 à 20, **caractérisée en ce que** R³ correspond à un ou plusieurs substituants qui sont choisis dans le groupe comprenant : le 9-fluorénylméthylcarbamate, CO₂CH₂CCl₃, CO₂CH₂CH₂Ph (Ph = phényle), CO₂C(CH₃)CHBr₂, CO₂C(CH₃)₂CCl₃, CO₂C(CH₃)₃, N-hydroxypipéridinylcarbamate, CO₂CH₂Ph (Ph = phényle), CO₂CH₂-*p*-CH₃OC₆H₄, CO₂CH₂-p-NO₂C₆H₄, CHO; COCH₃, COCH₂Cl, COCCl₃, COCF₃, COC₆H₅, phtalimide, dithiasuccinimide, N-5-dibenzosubéryl-amine, N-1,1-diméthylthiométhylène, N-benzylidène, N-1,3-dithiolan-2-ylidène, N-diphénylméthylène, =CHN(CH₃)₂ et =CN(CH₂C₆H₅)₂.

22. Utilisation selon l'une des revendication 16 à 21, **caractérisée en ce que** R⁴ correspond à un ou plusieurs substituants qui sont choisis dans le groupe comprenant CH₃, C₂H₅, CH₂OCH₃, CH₂SCH₃, CH₂OCH₂C₆H₅, CH₂CCl₃, C(CH₃)₃, CH₂C₆H₅, CH₂C₆H₂-2,4,6-(CH₃)₃.

23. Composé de L-énantiomère, **caractérisé par** la formule structurelle représentée ci-après : 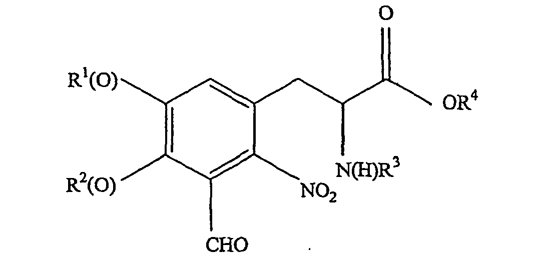 dans laquelle R¹ et R² sont des groupes protecteurs appropriés des groupes OH ;
R³ correspond à un groupe protecteur approprié pour une fonction amino ;
R⁴ correspond à un groupe protecteur d'une fonction carboxyle.

24. Composition de L-énantiomère selon la revendication 23, **caractérisée en ce que** R¹ et R² sont choisis respectivement dans le groupe comprenant : H, CH₃, CH₂OCH₃, CH₂OCH₂(C₆H₅), CH₂SCH₃, CH₂SCH₂(C₆H₅), CH₂OCH₂Cl, CH₂OCH₂Br, CH₂COC₆H₃-3,4-Cl, CH₂COC₆H₃-2,6-Cl₂, CH₂=CH₂, CH(CH₃)₂, c-C₆H₁₁, C(CH₃)₃, CH₂C₆H₅, 2,6-(CH₃)₂C₆H₃CH₂, 4-CH₃OCH₄CH₂, o-NO₂-C₆H₄CH₂, (CH₃)₂NCOC₆H₄CH₂, COCH₃, COC₆H₅, CO₂CH₃, COOCH₂CCl₃, CONHPh (Ph=phényle) et CONH-*i*-Bu (Bu = butyle).

25. Composé de L-énantiomère selon la revendication 23, **caractérisé en ce que** R¹ et R² correspondent à un acétal cyclique qui est choisi de préférence dans le groupe comprenant : les méthylène-acétal, diméthylène-acétal, cyclohexylidène-acétal, diphénylméthylène-acétal, éthoxyméthylène-acétal et un ester d'acide borique cyclique.

26. Composé de L-énantiomère selon des revendications précédentes, **caractérisé en ce que** R³ correspond à un ou plusieurs substituants qui sont choisis dans le groupe comprenant : le 9-fluorénylméthylcarbamate, CO₂CH₂CCl₃, CO₂CH₂CH₂Ph (Ph = phényle), CO₂C(CH₃)CHBr₂, CO₂C(CH₃)₂CCl₃, CO₂C(CH₃)₃, N-hydroxypipéridinylcarbamate, CO₂CH₂Ph (Ph = phényle), CO₂CH₂-*p-*CH₃OC₆H₄, CO₂CH₂-*p*-NO₂C₆H₄, CHO ; COCH₃, COCH₂Cl, COACH, COCF₃, COC₆H₅, phtalimide, dithiasuccinimide, N-5-dibenzosubéryl-amine, N-1,1-diméthylthiométhylène, N-benzylidène, N-1,3-dithiolan-2-ylidène, N-diphénylméthylène, =CHN(CH₃)₂ et =CN(CH₂C₆H₅)₂.

27. Composé de L-énantiomère selon l'une des revendications précédentes, **caractérisé en ce que** R⁴ correspond à un ou plusieurs substituants qui sont choisis dans le groupe comprenant CH₃, C₂H₅, CH₂OCH₃, CH₂SCH₃, CH₂OCH₂C₆H₅, CH₂CCl₃, C(CH₃)₃, CH₂C₆H₅, CH₂C₆H₂-2,4,6-(CH₃)₃.
